# DEMANDE DE BREVET EUROPEEN

(11) **EP 2 251 340 A1**
(43) Date de publication de la demande: **17.11.2010**
(21) Numéro de dépôt: 10173683.3
(22) Date de dépôt: 08.07.2004
(51) Int. Cl.: C07D 471/04, A61K 31/437, A61P 35/00

(54) **Tetrahydro-1h-pyrazolo[3,4-c] pyridines substituees, compositions les contenant et utilisation**

(30) Priorité: 10.07.2003 FR 0308442; 10.07.2003 FR 0308441
(62) Demande divisionnaire de: 04767613.5
(71) Demandeur: Aventis Pharma S.A., 92160 Antony (FR)
(72) Inventeur: Thompson, Fabienne, 75012, PARIS (FR); Mailliet, Patrick, 94120, FONTENAY-SOUS-BOIS (FR); Damiano, Teresa, 75013, PARIS (FR); Cherrier, Marie-Pierre, 94200, IVRY SUR SEINE (FR); Clerc, François, 92160, ANTONY (FR); Halley, Frank, 92310, SEVRES (FR); Bouchard, Hervé, 94320, THIAIS (FR); Gauzy-Lazo, Laurence, 75014, Paris (FR); Baudoin, Bernard, 92370, CHAVILLE (FR); Souaille, Catherine, 94600, CHOISY-LE-ROI (FR); Viviani, Fabrice, 95380, Louvres (FR); Tabart, Michel, 91290, LA NORVILLE (FR)
(74) Mandataire: Blot, Philippe Robert Emile

(57) **Abrégé**

La présente invention concerne notamment de nouvelles tétrahydro-1*H*-pyrazoio[3,4-c]pyridines substituées ayant une activité thérapeutique, utilisables en particulier en oncologie.

## Description

La présente invention concerne de nouveaux composés chimiques, particulièrement de nouvelles tétrahydro-1*H*-pyrazolo[3,4-*c*]pyridines, des compositions les contenant, et leur utilisation comme médicaments.

Plus particulièrement, l'invention concerne de nouvelles tétrahydro-1*H-*pyrazolo[3,4-*c*]pyridines présentant une activité anticancéreuse, et en particulier une activité inhibitrice de kinases, notamment Tie2.

Seules quelques tétrahydro-1*H*-pyrazolo[3,4-*c*]pyridines sont connues.

Ainsi, WO 02/012442 divulgue des tétrahydro-1*H*-pyrazolo[3,4-*c*]pyridines substituées en position 5 par un groupement amino, éventuellement substitué. Ces produits sont utiles pour le traitement du cancer et d'autres maladies liées à une prolifération cellulaire.

P. Krogsgaard-Larsen et al. dans Eur. J. Med. Chemical - Chim. Ther. (1979), 14(2), p.157-164 divulgue deux tétrahydro-1*H*-pyrazolo[3,4-c]pyridines substituées en position 3 par un groupement hydroxy.

WO 96/12720 revendique des tétrahydro-1*H*-pyrazolo[3,4-*c*]pyridines substituées en position 3 par des substituants choisis parmi H, alkyle, alkylène, cycloalkyle, et méthylènecycloalkyle, et en positions 1 et 6 par des substituants variés. Ces produits sont décrits comme inhibiteurs (i) de phosphodiestérase de type IV (PDE-IV), et (ii) de facteur de nécrose tumorale (TNF), et sont, de ce fait, considérés utiles dans le traitement de maladies inflammatoires. Aucun exemple de composé selon l'invention n'est divulgué.

Des tentatives pour obtenir des inhibiteurs efficaces de Tie2 ont déjà abouti dans le passé (voir à ce propos, par exemple, WO 98/02434 ; WO 98/41525 ; WO 99/10325; WO 99/17770; WO 99/54286; WO 99/21859 ; WO 99/55335 ; WO 00/17202 ; WO 00/17203 ; WO 00/27822 ; WO 00/75139 ; WO 01/37835 ; WO 01/57008 ; WO 01/72751 ; WO 02/060382 ; WO 02/076396 ; WO 02/076463; WO 02/076954; WO 02/076984; WO 02/076985 ; WO 02/080926 ; WO 03/004488).

Toutefois, aucun de ces documents ne divulgue de dérivés de 4,5,6,7-tétrahydro-1*H*-pyrazolo[3,4-*c*]pyridines telles que définies plus loin, présentant une activité à l'encontre de kinases, en particulier Tie2.

A cet effet, les produits conformes à l'invention selon son premier aspect répondent à la formule (I) suivante : 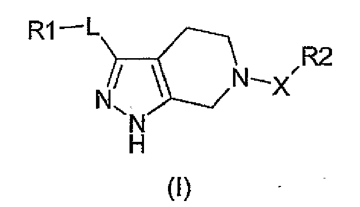 et ses tautomères, dans laquelle :
L est choisi parmi liaison, CH₂, CO, SO₂, CONH, COO, NHCO, NH, NHSO₂, SO₂NH, NHCONH, CH₂NH, NHCH₂,
X est choisi parmi liaison, CH₂, CO, SO₂, CONH, COO;
R1 est choisi parmi OH, H, alkyle, cycloalkyle, hétérocyclyle, aryle, hétéroaryle, éventuellement substitué, et, lorsque X est liaison, alors R1 peut aussi être halogène;
R2 est H ou choisi parmi alkyle, alkylène, cycloalkyle, hétérocyclyle, aryle, hétéroaryle, éventuellement substitué ;
les substituants étant indépendamment choisis parmi R3, O-R3, halogène, NO₂, SO₂-R3, CO-R3, SO2NH-R3, CONH-R3, N-(R3)₂, NHCO-R3, NHSO₂-R3, NHCONH-R3, NHSO₂NH-R3, OCO-R3, COO-R3, OSO₂-R3, SO₂O-R3, OCONH-R3, OSO₂NH-R3, où chaque R3 est indépendamment choisi parmi H, alkyle, cycloalkyle, alcènyle, aryle, hétéroaryle, hétérocyclyle, éventuellement substitué par halogène, aryle, hétéroaryle, R4, OR4, N(R4)₂, chaque R4 étant indépendamment choisi parmi H, C1-C4 alkyle, et C1-C4 alkyle halogéné.

Des produits conformes à l'invention selon son premier aspect sont plus particulièrement choisis parmi des produits de formule générale (II) suivante : 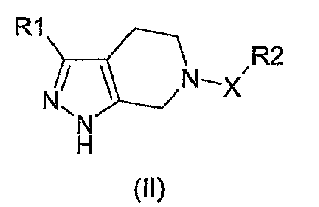 et ses tautomères, dans laquelle :
X est choisi parmi liaison, CH₂, CO, SO₂, CONH, COO ;
R1 est choisi parmi alkyle, cycloalkyle, hétérocyclyle, aryle, hétéroaryle, éventuellement substitué ;
R2 est H ou choisi parmi alkyle, alkylène, cycloalkyle, hétérocyclyle, aryle, hétéroaryle, éventuellement substitué ;
les substituants étant indépendamment choisis parmi R3, O-R3, halogène, NO₂, SO₂-R3, CO-R3, SO2NH-R3, CONH-R3, N-(R3)₂, NHCO-R3, NHSO₂-R3, NHCONH-R3, NHSO₂NH-R3, OCO-R3, COO-R3, OSO₂-R3, SO₂O-R3, OCONH-R3, OSO₂NH-R3, où chaque R3 est indépendamment choisi parmi H, alkyle, cycloalkyle, alcènyle, aryle, hétéroaryle, hétérocyclyle, éventuellement substitué par halogène, aryle, hétéroaryle, OR4, N(R4)₂, où chaque R4 est indépendamment choisi parmi H, C1-C4 alkyle.

Des produits conformes à l'invention selon son premier aspect sont plus particulièrement choisis parmi des produits de formule générale (III) suivante : 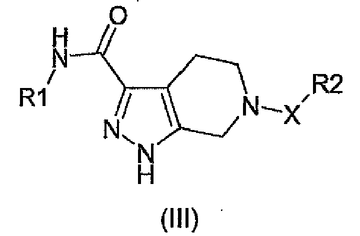 et ses tautomères, dans lesquels :
X est choisi parmi liaison, CH₂, CO, SO₂, CONH, COO ;
R1 est choisi parmi alkyle, cycloalkyle, hétérocyclyle, aryle, hétéroaryle, éventuellement substitué ;
R2 est H ou choisi parmi alkyle, alkylène, cycloalkyle, hétérocyclyle, aryle, hétéroaryle, éventuellement substitué ;
   dans lequel les substituants sont indépendamment choisis parmi R3, O-R3, halogène, NO₂, SO₂-R3, CO-R3, S02NH-R3, CONH-R3, N-(R3)₂, NHCO-R3, NHSO₂-R3, NHCONH-R3, NHSO₂NH-R3, OCO-R3, COO-R3, OSO₂-R3, SO₂O-R3, OCONH-R3, OSO₂NH-R3, dans lequel chaque R3 est indépendamment choisi parmi H, alkyle, cycloalkyle, alcènyle, aryle, hétéroaryle, hétérocyclyle, éventuellement substitué par halogène, aryle, hétéroaryle, OR4, N(R4)₂, et dans lequel chaque R4 est indépendamment choisi parmi H, C1-C4 alkyle.

Un produit conforme à l'invention est avantageusement choisi parmi les produits selon son premier aspect, dans lesquels R1 est hérétoaryle, éventuellement substitué, dans lequel un hétéroaryle préféré est choisi parmi benzimidazolyle, indolyle, pyrrolyle, éventuellement substitué par halogène, R4, O-R4.

Plus particulièrement, un hétéroaryle préféré est choisi parmi benzimidazol-2-yle, indol-2-yle, pyrrol-2-yle, éventuellement substitué par halogène, R4, OR4.

Un produit conforme à l'invention selon son premier aspect a avantageusement un substituant R2 choisi parmi phényle, pyridyle, thiényle, C1-C4 alkyle, et C3-C7 cycloalkyle, éventuellement substitué.

X peut être avantageusement choisi parmi CO et SO₂.

Un produit conforme à l'invention selon son premier aspect est avantageusement choisi parmi les produits de formule (I), dans laquelle R1 est H.

Un produit préféré est avantageusement choisi parmi les produits de formule (I) dans lesquels R1 est aryle substitué.

Selon un premier mode de réalisation préféré, un produit préféré est avantageusement choisi parmi les produits de formule générale (I) dans lesquels R1-L est R1-NH-CO, et plus préférentiellement dans les cas où R1 est H.
Très préférentiellement, et selon un second mode de réalisation préféré, un produit préféré est avantageusement choisi parmi (i) les produits de formule générale (I) ou (ii) de préférence les produits selon le premier mode de réalisation préféré, dans laquelle X est liaison, et dans laquelle R2 est choisi parmi aryle substitué et hétéroaryle substitué.

Selon un troisième mode de réalisation préféré, un produit plus préféré est choisi parmi les produits conformes à l'invention selon son second mode de réalisation, dans lequel R2 est choisi parmi
- aryle substitué par NHSO₂-R3 ou NHCONH-R3, et
- hétéroaryle substitué par NHSO₂-R3, ou NHCONH-R3.

Des produits selon le troisième mode de réalisation préféré sont avantageusement choisis parmi
- aryle substitué par NHSO₂-R3 ou NHCONH-R3, et
- hétéroaryle substitué par NHSO₂-R3, ou NHCONH-R3,
dans lequel aryle est phényle, et dans lequel hétéroaryle est choisi parmi pyridyle et pyrimidyle.

Selon un quatrième mode de réalisation, des produits selon le troisième mode de réalisation préféré sont avantageusement choisis parmi
- aryle substitué par NHSO₂-R3 ou NHCONH-R3, et
- hétéroaryle substitué par NHSO₂-R3, ou NHCONH-R3,
dans lesquels R3 est choisi parmi aryle substitué et hétéroaryle substitué,
dans lequel R3 est avantageusement substitué par un substituant sélectionné dans le groupe constitué par halogène, R4, OR4, N(R4)₂, dans lequel chaque R4 est indépendamment choisi parmi H, C1-C4 alkyle, et C1-C4 alkyle halogéné.

Selon un cinquième mode de réalisation, des produits selon le quatrième mode de réalisation préféré sont avantageusement choisis parmi: 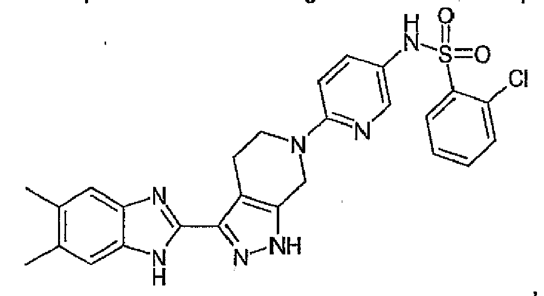 et

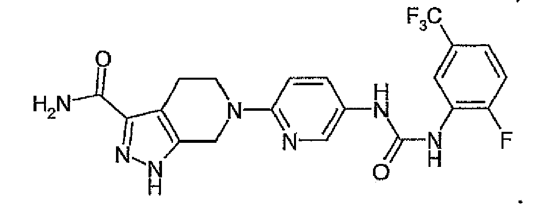

Un produit conforme à l'invention selon son premier aspect, peut se présenter sous forme :
1) racémique, ou
2) enrichie en un stéréoisomère, ou
3) enrichie en un énantiomère ;
   et être éventuellement salifié.

Selon un second aspect, l'invention concerne des compositions pharmaceutiques comprenant un produit tel que défini précédemment, en combinaison avec un excipient pharmaceutiquement acceptable.

Selon un troisième aspect, l'invention concerne l'utilisation d'un produit tel que défini précédemment, comme agent modulant l'activité d'une kinase. Une kinase préférée sera avantageusement choisie parmi Tie2 et KDR. Tie2 est plus préférée.

Selon son troisième aspect, l'invention concerne l'utilisation d'un produit tel que défini précédemment, pour la fabrication d'un médicament utile pour traiter un état pathologique, en particulier le cancer.

Des produits conformes à l'invention peuvent être obtenus par de méthodes bien connues de l'homme de l'art, en particulier en ce qui concerne les techniques de couplage entre un acide et une amine. Voir, par exemple, J. March, Advanced organic chemistry, (J. Wiley & Sons, ed.), quatrième édition, 1992.

Les produits de l'invention sont utiles comme agents inhibiteurs d'une réaction catalysée par une kinase. Tie2 est une kinase pour laquelle les produits de l'invention seront particulièrement utiles en tant qu'inhibiteurs. Ces produits peuvent aussi être utiles comme inhibiteurs d'autres kinases telles que KDR.

Des raisons pour lesquelles ces kinases sont choisies sont données ci- après :

### Tie2

Tie-2 (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïetine 1 ou Ang1) qui stimule l'autophosphoryiation du récepteur et la signalisation cellulaire [S. Davis et al (1996) Cell 87, 1161-1169] et l'antagoniste (angiopoïetine 2 ou Ang2) *[*P.C. Maisonpierre et al. (1997) Science 277, 55-60]*.* L'angiopoïetine 1 peut synergiser avec le VEGF dans les derniers stades de la néo- angiogénèse [AsaharaT. Circ. Res.(1998) 233-240]*.* Les expériences de knock-out et les manipulations transgéniques de l'expression de Tie2 ou de Ang1 conduisent à des animaux qui présentent des défauts de vascularisation *[*D.J. Dumont et al (1994) Genes Dev. 8, 1897-1909 *et* C. Suri (1996) Cell 87, 1171-1180]. La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P.C. Maisonpierre et al (1997) Science 277, 55-60]. Lin et al (1997) J. Clin. Invest. 100, 8: 2072-2078 *et* Lin P. (1998) PNAS 95, 8829-8834, ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénographes de tumeur du sein et de mélanome.

Les inhibiteurs de Tie2 peuvent être utilisés dans les situations où une néovascularisation se fait de façon inappropriée (c'est-à-dire dans la rétinopathie diabétique, l'inflammation chronique, le psoriasis, le sarcome de Kaposi, la néovascularisation chronique due à la dégénération maculaire, l'arthrite rhumatoïde, l'hémoanglome infantile et les cancers).

### KDR

KDR (Kinase insert Domain Receptor) aussi appelée VEGF-R2 (Vascular Endothelial Growth Factor Receptor 2), est exprimé uniquement dans les cellules endothéliales. Ce récepteur se fixe au facteur de croissance angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor : facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. Par conséquent, un inhibiteur sélectif de l'activité kinase du VEGF-R2 ne devrait démontrer que peu de toxicité.

En plus de ce rôle central dans le processus dynamique angiogénique, des résultats récents suggèrent que l'expression de VEGF contribue à la survie des cellules tumorales après des chimie- et radio-thérapies, soulignant la synergie potentielle d'inhibiteurs de KDR avec d'autres agents (Lee et al. Cancer Research, 2000, vol. 60, p.5565-5570).

### Partie expérimentale

### Méthode A : Analyse par LC/MS

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses Mass spectra a été réalisée sur une gamme de 180 à 800. Les données ont été analysées en utilisant le logiciel Micromass MassLynx. La séparation a été effectuée sur une colonne Hypersil BDS C18, 3 µm (50 x 4.6 mm), en éluant par un gradient linéaire de 5 à 90% d'acétonitrile contenant 0,05% (v/v) d'acide trifiluoroacëtique (TFA) dans l'eau contenant 0,05% (v/v) TFA en 3,5 mn à un débit de 1 mL/mn. Le temps total d'analyse, incluant la période de rééquilibration de la colonne, est de 7 mn.

### Méthode B : Purification par LC/MS:

Les produits ont été purifiés par LC/MS en utilisant un système Waters FractionsLynx composé d'une pompe à gradient Waters modèle 600, d'une pompe de régénération Waters modèle 515, d'une pompe de dilution Waters Reagent Manager, d'un auto-injecteur Waters modèle 2700, de deux vannes Rheodyne modèle LabPro, d'un détecteur à barrette de diodes Waters modèle 996, d'un spectromètre de masse Waters modèle ZMD et d'un collecteur de fractions Gilson modèle 204. Le système étant contrôlé par le logiciel Waters FractionLynx. La séparation a été effectuée alternativement sur deux colonnes Waters Symmetry (C₁₈, 5µM, 19x50 mm, référence catalogue 186000210), une colonne étant en cours de régénération par un mélange eau / acétonitrile 95/5 (v/v) contenant 0,07% (v/v) d'acide trifluoroacétique, pendant que l'autre colonne est en cours de séparation. L'élution des colonnes a été effectuée en utilisant un gradient linéaire de 5 à 95% d'acétonitrile contenant 0,07% (v/v) d'acide trifluoroacétique dans l'eau contenant 0,07% (v/v) d'acide trifluoroacétique, à un débit de 10 mL/mn. A la sortie de la colonne de séparation, un millième de l'effluent est séparé par un LC Packing Accurate, dilué à l'alcool méthylique à un débit de 0,5 mL/mn et envoyé vers les détecteurs, à raison de 75% vers le détecteur à barrette de diodes, et les 25% restants vers le spectromètre de masse. Le reste de l'effluent (999/1000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Na]⁺. Dans certains cas, dépendant des résultats de LC/MS analytique, quand un ion intense correspondant à [M+2H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺ et/ou [M+Na+H]⁺⁺ sont détectés.

### Méthode C : Analyse El

Les spectres de masse ont été réalisés en impact électronique (70eV) sur spectromètre Finnigan SSQ 7000.

### Méthode D : Analyse RMN

Les spectres RMN ont été réalisés sur spectromètre BRUKER Avance 300 et BRUKER Avance DRX 400.

### 4-(Diazo-éthoxycarbonyl-méthyl)-4-hydroxy-pipéridine-1-carboxylate de tert-butyle

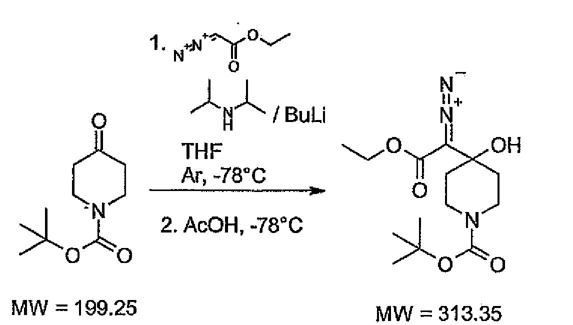

| | CAS | Nom | d | MM | éq | mmol | g | mL |
|---|---|---|---|---|---|---|---|---|
| 1 | 79099-07-3 | N-Boc-pipéridinone | | 199.25 | 1.00 | 50.19 | 10.00 | |
| 2 | 623-73- 4 | diazoacétate d'éthyle | 1.085 | 114.1 | 1.05 | 52.70 | 6.01 | 5.54 |
| 3 | 109-72-8 | BuLi 1,6 M hexane | | | 1.60 | 80.30 | | 50.19 |
| 4 | 108-18-9 | di-isopropyl amine | 0.720 | 101.19 | 1.60 | 80.30 | 8.13 | 11.29 |
| 5 | 109-99-9 | THF sur tamis moléculaire 4Å | | | 10 vol. | | | 500 |
| 6 | 64-19-7 | AcOH 100% | 1.050 | 60.05 | 5.00 | 250.94 | 15.07 | 14.35 |

Une solution fraîchement préparée de LDA ( préparé par addition goutte à goutte sous atmosphère inerte à -78°C de 50,19 mL de BuLi 1,6M dans l'hexane sur une solution de 11,29 mL de diisopropylamine dans 200 mL de THF sec) est ajoutée goutte à goutte sous atmosphère inerte à -78°C sur 10.0 g de N-Boc-pipéridinone en suspension et 5.54 mL de diazoacétate d'éthyle dans 300 mL de THF sec. Le mélange est agité à -78°C pendant 4 heures puis est décomposé à -78°C par 14,35 mL d'AcOH concentré. Le mélange obtenu est laissé à température ambiante pendant une nuit, puis le solvant est évaporé sous pression réduite jusqu'à 1/10^{ème} de son volume initial, dilué dans de l'oxyde de diisopropyle, et lavé 4 fois avec une solution saturée de NaHCO₃. La phase organique est séchée sur MgSO₄. Le sel hydraté est éliminé par filtration et le filtrat sec est concentré sous pression réduite pour fournir 15,12g d'une huile visqueuse jaune. LC/MS: RT = 2.84 [M+1]+ = 304.33. Le produit est utilisé tel quel pour l'étape suivante.

### 4-(Diazo)-éthoxycarbonyl-méthyl)-3,6-dihydro-2H-pyridine-1-carboxylate de tert-butyle

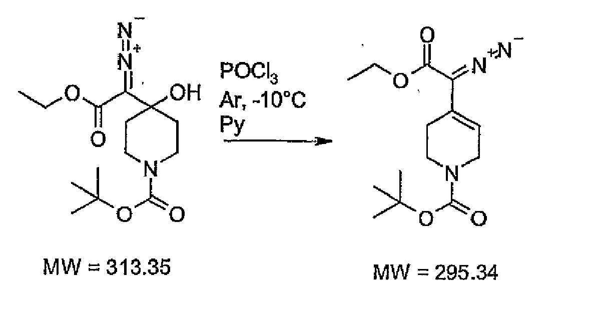

| | CAS | Nom | d | MM | éq | mmol | g | mL |
|---|---|---|---|---|---|---|---|---|
| 1 | | P-31391-106-4 | | 313.35 | 1 | 48.25 | 15.12 | |
| 2 | 10025-87-3 | POCl₃ | 1.67 | 153.33 | 2 | 96.51 | 14.80 | 8.86 |
| 3 | 110-86-1 | Pyridine (m.s. 4Å) | 0.983 | 79.1 | 20 | 965.06 | 76.34 | 77.66 |
| 4 | 108-20-3 | *i*Pr₂O | | | 5 vol | | | 250 |
| 5 | 1310-73-2 | NaOH 0.1M | | 40 | 1 | 48.25 | | 483 |

78,0 mL de pyridine sèche sont ajoutés sur une solution de 15.12 g de 4-(Diazo-éthoxycarbonyl-méthyl)-4-hydroxy-pipéridine-1-carboxylate de tert-butyle dans 250 mL de *i*Pr₂O. Le mélange est refroidi à -10°C et 8,86 mL de POCl₃ sont ajoutés lentement sous agitation vigoureuse. Le mélange est alors laissé remonter à température ambiante sous agitation pendant 12 heures. Le mélange réactionnel est décomposé par 500 mL d'une solution de NaOH 0.1 M, puis est extraite 3 fois avec AcOEt. La phase organique est lavée avec une solution saturée de NaCl et séchée sur MgSO₄. Le sel hydraté est éliminé par filtration et le filtrat sec est concentré sous pression réduite jusqu'à 1/10^{ème} de son volume initial. LC/MS: RT = 4.57 [M+1]+ = 296.31. Le produit est utilisé tel quel pour l'étape suivante.

### 2,4,5,7-Tétrahydro-pyrazolo[3,4-c]pyddyl-3,6-dicarboxylate de 6-tert-butyle 3-éthyle.

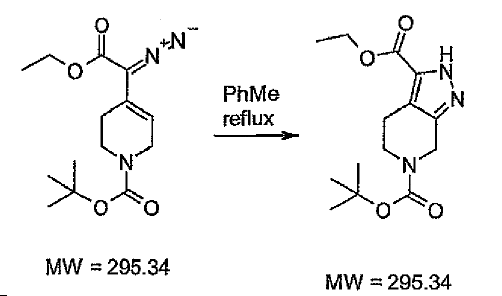

| | **CAS** | **Nom** | **d** | **MM** | **éq** | **mmol** | **mg** | **mL** |
|---|---|---|---|---|---|---|---|---|
| **1** | | P-31391-120-4 | | 285.35 | 1.0 | 48.25 | | |
| **2** | 108-83-3 | PhMe | | | | | | 150 |

La solution de 4-(Diazo-éthoxycarbonyl-méthyl)- 3,6-dihydro-2H-pyridyl-1-carboxylate de tert-butyle dans Py/AcOEt obtenue à l'étape précédente est additionnée goutte à goutte sur 150 mL de toluène au reflux. L'azéotrope Py/PhMe est distillé à une vitesse équivalente à la vitesse d'addition. 1 heure après la fin de l'addition, la solution est laissée refroidir à température ambiante, le solvant est évaporé sous pression réduite et le produit brut obtenu (15.05 g) est purifié par flash-chromatographie (SiO₂, CH₂Cl₂/MeOH 1% NH₃ 7M_{(MeOH)} 40:1 puis 30:1 puis 20:1). Le solvant est évaporé et 10,05 g (71% sur 3 étapes) d'un solide noir sont obtenus : LC/MS: RT = 3.88 [M+1]+ = 296.27

### Acide (2,4,5,7-Tétrahydro-pyrazolo[3,4-c]pyridyl-6-carboxylate de tert-butyle)-3-carboxylique

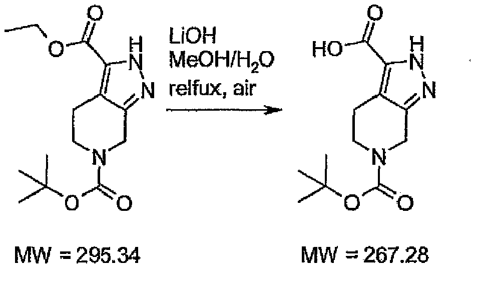

| | CAS | Nom | d | MM | éq | mmol | g | mL |
|---|---|---|---|---|---|---|---|---|
| 1 | | P-31391-123-1 | | 295.34 | 1.0 | 34.03 | 10.05 | |
| 2 | 1310-66-3 | LiOH•H₂O | | 41.96 | 1.1 | 37.43 | 1.57 | |
| 3 | 67-56-1 | MeOH | | | 10 vol. | | | 375 |
| 4 | 7732-18-5 | H₂O | | | 1vol | | | 38 |

1,57 g de LiOH et 38 mL d'eau sont ajoutés à une solution de de 10,05 g de 2,4,5,7-Tétrahydro-pyrazolo[3,4-c]pyridyl-3,6-dicarboxylate de 6-tert-butyle 3-éthyle dans 375 mL de MeOH. Le mélange obtenu est chauffé au reflux pendant une nuit. La solution est refroidie à température ambiante, puis est acidifiée à pH = 2 avec 50 mL d'une solution de HCl 1 M. La solution est ensuite extraite 4 fois par AcOEt. La phase organique est lavée par une solution saturée de NaCl puis séchée sur Na₂SO₄. Le sel obtenu est éliminé par filtration, et le solvant est évaporé sous pression réduite pour produire 8,90g (98%) d'un solide blanc. LC/MS: RT = 3.21 [M+1]+ = 268.23.

### Préparation d'une bibliothèque de produits :

### 3-(alky/carbamoy/, arylcarbamoyl, hétéroarylcarbamoyl, etc...)-2,4,5,7-tétrahydro-pyrazolo[3,4-c]pyridine-6-carboxylate de tert-butyle

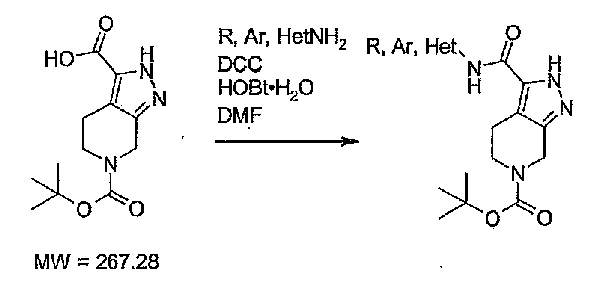

| | CAS | Nom | d | MM | éq | mmol | g | mL |
|---|---|---|---|---|---|---|---|---|
| 1 | | P-31391-031-5 | | 267.28 | 1.0 | 3.741 | 1.00 | |
| | | amine | | | 2.0 | 6.744 | | |
| 2 | 538-75-0 | DCC | | 206.33 | 1.0 | 3.741 | 0.772 | |
| 3 | 2592-95-2 | HOBt•H₂O | | 155.13 | 1.5 | 5.612 | 0.859 | |
| 4 | 68-12-2 | DMF | | | 5 vol. | | | 19 |

### Méthode générale:

Du DCC et de l'HOBt.H₂O en solution dans le DMF avec 2 éq. d'amine (R, Ar, ou Het)-NH₂ sont ajoutés à une solution de 1 éq. d'acide (2,4,5,7-Tétrahydro-pyrazolo[3,4-c]pyridyl-6-carboxylate de tert-butyle)-3-carboxylique dans le DMF et le mélange est agité à température ambiante pendant 3h. Le solvant est évaporé sous pression réduite à 35°C pendant une nuit. Le produit brut obtenu est purifié parflash-chromatographie (SiO₂, CH₂Cl₂/MeOH 1 % NH₃ 7M_{(MeOH)} 20:1 puis 10:1 puis 5:1, en fonction des produits).

Liste des amines R1-NH₂ utilisées (Tableau 1) [Remarque : R1-NH₂ = (R, Ar, ou Het)-NH₂]:

**Tableau 1**

| Numéro de référence de l'amine | Structure |
|---|---|
| 1 | 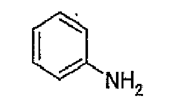 |
| 2 | 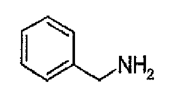 |
| 3 | 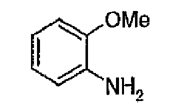 |
| 4 | 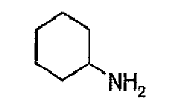 |
| 5 | 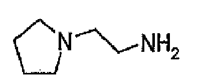 |
| 6 | 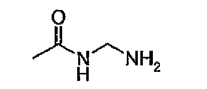 |
| 7 | 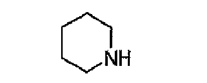 |
| 8 | 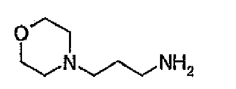 |
| 9 | 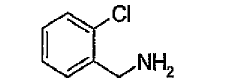 |
| 10 | 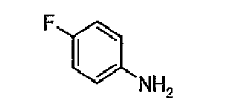 |
| 11 | 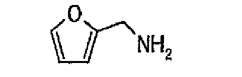 |
| 12 | 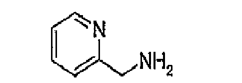 |
| 13 | 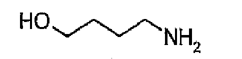 |
| 14 | 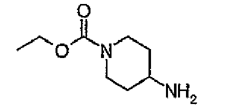 |
| 15 | 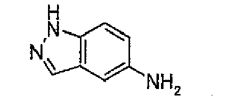 |

### Trifluoroacétate de 3-(alkylcarbamoyl, arylcarbamoyl, hétéroarylcarbamoyl, etc...)-4,5,6,7-tétrahydro-2H-pyrazolo[3,4-c]pyridin-6-ium

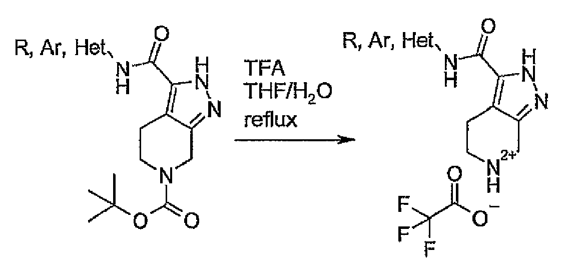

### Méthode générale :

16 éq de TFA sont ajoutés sur 1 éq de 3-(alkylcarbamoyl, arylcarbamoyl, hétéroarylcarbamoyl, etc...)-2,4,5,7-tétrahydro-pyrazolo[3,4-c]pyridine-6-carboxylate de tert-butyle dans une solution 1 :1 THF/eau et la solution est chauffée au reflux pendant 2h. La solvant est évaporé sous pression réduite et l'huile visqueuse recueillie est séchés sous vide pendant une nuit. Le produit ainsi obtenu est utilisé sans purification à l'étape ultérieure.

### - 6-(Alkyl, aryl, hétéroaryl, etc...)carbonyl-4,5,6,7-tétmhydro-2H-pymazolo[3,4-c]pyridine-3-(alkyl, aryrl, hetyl, etc.)-amide

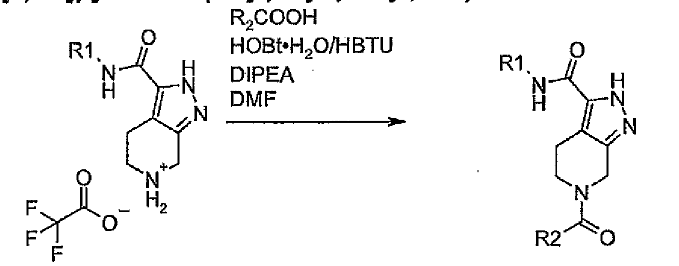

### Méthode générale :

Sur une solution de 1 éq. de trifluoroacétate de 3-(alkylcarbamoyl, arylcarbamoyl, hétéroarylcarbamoyl, etc...)-4,5,6,7-tétrahydro-2H-pyrazolo[3,4-c]pyridin-6-ium dans le DMF sont ajoutés, dans l'ordre, une solution de HOBt•H₂O 2.5 M (2 éq.) dans le DMF, une solution de HBTU 0.833 M dans le DMF (2 éq.), une solution de DIPEA 2.5 M (4 éq.) dans le DMF et une suspension ou une solution à une concentration appropriée d'un R₂COOH (2 éq.) dans le DMF. Les solutions sont agitées une nuit à température ambiante, puis sont acidifiées avec 100µL d'AcOH 100%, filtrées et purifiées par LC/MS préparative.

### Liste des acides R₂COOH utilisés (tableau 2) :

**Tableau 2**

| Numéro de référence de l'acide | Nomenclature |
|---|---|
| 1 | Acide 1-phényl-1-cyclopropylcarboxylique |
| 2 | Acide acétique |
| 3 | Acide propiolique |
| 4 | Acide crotonique |
| 5 | Acide vinylacétique |
| 6 | Acide pyruvique |
| 7 | Sarcosine |
| 8 | Acide méthoxyacétique |
| 9 | Acide lactique |
| 10 | Acide 3,3-diméthylacrylique |
| 11 | Acide cyclopropylacétique |
| 12 | Acide valérique |
| 13 | N,N-diméthylglycine |
| 14 | Acide 3-mercaptopropionique |
| 15 | Acide (méthylthio)acétique |
| 16 | Acide pyrrole-2-carboxylique |
| 17 | Acide 1-cyanocyclopropane-carboxylique |
| 18 | Acide 2-furoïque |
| 19 | Acide 4-pyrazolecarboxylique |
| 20 | Acide imidazole-4-carboxylique |
| 21 | Acide 1-cyclopentènecarboxylique |
| 22 | Acide |
| 23 | Acide acétoxyacétique |
| 24 | Acide hydantoïque |
| 25 | Acide benzoïque |
| 26 | Acide nicotinique |
| 27 | Acide 2-pyrazinecarboxylique |
| 28 | Acide o-toluique |
| 29 | Acide phénylacétique |
| 30 | Acide salicylique |
| 31 | Acide 2-fluorobenzoïque |
| 32 | Acide 3-cyanobenzoïque |
| 33 | Acide 4-vinylbenzoïque |
| 34 | Acide 2-phénylpropionique |
| 35 | Acide N-méthylanthranilique |
| 36 | Acide 2-méthoxybenzoïque |
| 37 | Acide 2-hydroxyphénylacétique |
| 38 | Acide 4-hydroxyméthylbenzoïque |
| 39 | Acide 2-fluorophénylbenzoïque |
| 40 | Acide 2,6-difluorobenzoïque |
| 41 | Acide indole-3-carboxylique |
| 42 | Acide 3,5-diméthylphénylacétique |
| 43 | Acide 3-(diméthylamino)benzoïque |
| 44 | Acide pipéronylique |
| 45 | Acide DL-tropique |
| 46 | Acide 3-méthoxyphénylacétique |
| 47 | Acide 3-méthoxysalicylique |
| 48 | Acide 4-(méthylthio)benzoïque |
| 49 | Acide 2-chlorophénylacétique |
| 50 | Acide 2-naphtoïque |
| 51 | Acide 2-chloro-6-fluorobenzoïque |
| 52 | Acide 1-méthylindole-3-carboxylique |
| 53 | Acide 3-acétamidobenzoïque |
| 54 | Acide 4-(diméthylamino)salicylique |
| 55 | Acide 2,3-diméthoxybenzoïque |
| 56 | Acide 4-chiorophénylpropionique |
| 57 | Acide 2-chloromandélique |
| 58 | Acide 2-chloro-6-fluoro-phénylacétique |
| 59 | Acide 1-phényl-1-cyclopentanecarboxylique |
| 60 | Acide 2,6-dichlorobenzoïque |
| 61 | Acide 3-méthyl-2-phénylvalérique |
| 62 | Acide 4-phénylbenzoïque |
| 63 | Acide 2-chloro-4-nitrobenzoïque |
| 64 | Acide 2-benzylbenzoïque |
| 65 | Acide 2-phénoxybenzoïque |
| 66 | Acide 2-éthoxy-1-naphtoïque |
| 67 | Acide 4-(4-N-propylphényl)-benzoïque |
| 68 | Acide 3,5-dibromosalicylique |
| 69 | Acide 2,6-dichloro-phénylacétique |
| 70 | Acide cyanoacétique |

### Résultats

Les produits suivants sont préparés selon le procédé décrit plus haut. 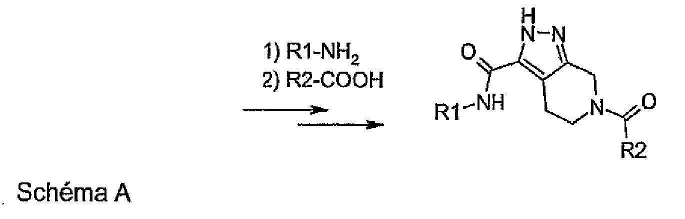 Afin de simplifier la représentation des produits dans le tableau 3 qui suit, le noyau pyrazolopipéridine présenté sur le schéma A est symbolisé par la lettre H, les amines R1-NH₂ qui sont liées à H sont symbolisées par la lettre B suivie d'un numéro allant de 1 à 15, correspondant aux produits listés dans le tableau 1, les acides R2-COOH qui sont liés à H sont symbolisés par la lettre A suivie d'un numéro de 1 à 70, correspondant aux produits listés dans le tableau 2.

Ainsi un produit noté A1-H-B1 correspond à la structure suivante :

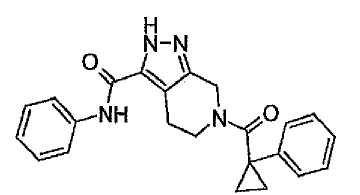

**Tableau 3**

| | | | | | | |
|---|---|---|---|---|---|---|
| A1-H-B1 | A2-H-B1 | A3-H-B1 | A4-H-B1 | A5-H-B1 | A6-H-B1 | A7-H-B1 |
| A1-H-B2 | A2-H-B2 | A3-H-B2 | A4-H-B2 | A5-H-B2 | A6-H-B2 | A7-H-B2 |
| A1-H-B3 | A2-H-B3 | A3-H-B3 | A4-H-B3 | A5-H-B3 | A6-H-B3 | A7-H-B3 |
| A1-H-B4 | A2-H-B4 | A3-H-B4 | A4-H-B4 | A5-H-B4 | A6-H-B4 | A7-H-B4 |
| A1-H-B5 | A2-H-B5 | A3-H-B5 | A4-H-B5 | A5-H-B5 | A6-H-B5 | A7-H-B5 |
| A1-H-B6 | A2-H-B6 | A3-H-B6 | A4-H-B6 | A5-H-B6 | A6-H-B6 | A7-H-B6 |
| A1-H-B7 | A2-H-B7 | A3-H-B7 | A4-H-B7 | A5-H-B7 | A6-H-B7 | A7-H-B7 |
| A1-H-B8 | A2-H-B8 | A3-H-B8 | A4-H-B8 | A5-H-B8 | A6-H-B8 | A7-H-B8 |
| A1-H-B9 | A2-H-B9 | A3-H-B9 | A4-H-B9 | A5-H-B9 | A6-H-B9 | A7-H-B9 |
| A1-H-B10 | A2-H-B10 | A3-H-B10 | A4-H-B10 | A5-H-B10 | A6-H-B10 | A7-H-B10 |
| A1-H-B11 | A2-H-B11 | A3-H-B11 | A4-H-B11 | A5-H-B11 | A6-H-B11 | A7-H-B11 |
| A1-H-B12 | A2-H-B12 | A3-H-B12 | A4-H-B12 | A5-H-B12 | A6-H-B12 | A7-H-B12 |
| A1-H-B13 | A2-H-B13 | A3-H-B13 | A4-H-B13 | A5-H-B13 | A6-H-B13 | A7-H-B13 |
| A1-H-B14 | A2-H-B14 | A3-H-B14 | A4-H-B14 | A5-H-B14 | A6-H-B14 | A7-H-B14 |
| A1-H-B15 | A2-H-B15 | A3-H-B15 | A4-H-B15 | A5-H-B15 | A6-H-B15 | A7-H-B15 |
| A8-H-B1 | A9-H-B1 | A10-H-B1 | A11-H-B1 | A12-H-B1 | A13-H-B1 | A14-H-B1 |
| A8-H-B2 | A9-H-B2 | A10-H-B2 | A11-H-B2 | A12-H-B2 | A13-H-B2 | A14-H-B2 |
| A8-H-B3 | A9-H-B3 | A10-H-B3 | A11-H-B3 | A12-H-B3 | A13-H-B3 | A14-H-B3 |
| A8-H-B4 | A9-H-B4 | A10-H-B4 | A11-H-B4 | A12-H-B4 | A13-H-B4 | A14-H-B4 |
| A8-H-B5 | A9-H-B5 | A10-H-B5 | A11-H-B5 | A12-H-B5 | A13-H-B5 | A14-H-B5 |
| A8-H-B6 | A9-H-B6 | A10-H-B6 | A11-H-B6 | A12-H-B6 | A13-H-B6 | A14-H-B6 |
| A8-H-B7 | A9-H-B7 | A10-H-B7 | A11-H-B7 | A12-H-B7 | A13-H-B7 | A14-H-B7 |
| A8-H-B8 | A9-H-B8 | A10-H-B8 | A11-H-B8 | A12-H-B8 | A13-H-B8 | A14-H-B8 |
| A8-H-B9 | A9-H-B9 | A10-H-B9 | A11-H-B9 | A12-H-B9 | A13-H-B9 | A14-H-B9 |
| A8-H-B10 | A9-H-B10 | A10-H-B10 | A11-H-B10 | A12-H-B10 | A13-H-B10 | A14-H-B10 |
| A8-H-B11 | A9-H-B11 | A10-H-B11 | A11-H-B11 | A12-H-B11 | A13-H-B11 | A14-H-B11 |
| A8-H-B12 | A9-H-B12 | A10-H-B12 | A11-H-B12 | A12-H-B12 | A13-H-B12 | A14-H-B12 |
| A8-H-B13 | A9-H-B13 | A10-H-B13 | A11-H-B13 | A12-H-B13 | A13-H-B13 | A14-H-B13 |
| A8-H-B14 | A9-H-B14 | A10-H-B14 | A11-H-B14 | A12-H-B14 | A13-H-B14 | A14-H-B14 |
| A8-H-B15 | A9-H-B15 | A10-H-B15 | A11-H-B15 | A12-H-B15 | A13-H-B15 | A14-H-B15 |
| A15-H-B1 | A16-H-B1 | A17-H-B1 | A18-H-B1 | A19-H-B1 | A20-H-B1 | A21-H-B1 |
| A15-H-B2 | A16-H-B2 | A17-H-B2 | A18-H-B2 | A19-H-B2 | A20-H-B2 | A21-H-B2 |
| A15-H-B3 | A16-H-B3 | A17-H-B3 | A18-H-B3 | A19-H-B3 | A20-H-B3 | A21-H-B3 |
| A15-H-B4 | A16-H-B4 | A17-H-B4 | A18-H-B4 | A19-H-B4 | A20-H-B4 | A21-H-B4 |
| A15-H-B5 | A16-H-B5 | A17-H-B5 | A18-H-B5 | A19-H-B5 | A20-H-B5 | A21-H-B5 |
| A15-H-B6 | A16-H-B6 | A17-H-B6 | A18-H-B6 | A19-H-B6 | A20-H-B6 | A21-H-B6 |
| A15-H-B7 | A16-H-B7 | A17-H-B7 | A18-H-B7 | A19-H-B7 | A20-H-B7 | A21-H-B7 |
| A15-H-B8 | A16-H-B8 | A17-H-B8 | A18-H-B8 | A19-H-B8 | A20-H-B8 | A21-H-B8 |
| A15-H-B9 | A16-H-B9 | A17-H-B9 | A18-H-B9 | A19-H-B9 | A20-H-B9 | A21-H-B9 |
| A15-H-B10 | A16-H-B10 | A17-H-B10 | A18-H-B10 | A19-H-B10 | A20-H-B10 | A21-H-B10 |
| A15-H-B11 | A16-H-B11 | A17-H-B11 | A18-H-B11 | A19-H-B11 | A20-H-B11 | A21-H-B11 |
| A15-H-B12 | A16-H-B12 | A17-H-B12 | A18-H-B12 | A19-H-B12 | A20-H-B12 | A21-H-B12 |
| A15-H-B13 | A16-H-B13 | A17-H-B13 | A18-H-B13 | A19-H-B13 | A20-H-B13 | A21-H-B13 |
| A15-H-B14 | A16-H-B14 | A17-H-B14 | A18-H-B14 | A19-H-B14 | A20-H-B14 | A21-H-B14 |
| A15-H-B15 | A16-H-B15 | A17-H-B15 | A18-H-B15 | A19-H-B15 | A20-H-B15 | A21-H-B15 |
| A22-H-B1 | A23-H-B1 | A24-H-B1 | A25-H-B1 | A26-H-B1 | A27-H-B1 | A28-H-B1 |
| A22-H-B2 | A23-H-B2 | A24-H-B2 | A25-H-B2 | A26-H-B2 | A2T-H-B2 | A28-H-B2 |
| A22-H-B3 | A23-H-B3 | A24-H-B3 | A25-H-B3 | A26-H-B3 | A27-H-B3 | A28-H-B3 |
| A22-H-B4 | A23-H-B4 | A24-H-B4 | A25-H-B4 | A26-H-B4 | A27-H-B4 | A28-H-B4 |
| A22-H-B5 | A23-H-B5 | A24-H-B5 | A25-H-B5 | A26-H-B5 | A27-H-B5 | A28-H-B5 |
| A22-H-B6 | A23-H-B6 | A24-H-B6 | A25-H-B6 | A26-H-B6 | A27-H-B6 | A28-H-B6 |
| A22-H-B7 | A23-H-B7 | A24-H-B7 | A25-H-B7 | A26-H-B7 | A27-H-B7 | A28-H-B7 |
| A22-H-B8 | A23-H-B8 | A24-H-B8 | A25-H-B8 | A26-H-B8 | A27-H-B8 | A28-H-B8 |
| A22-H-B9 | A23-H-B9 | A24-H-B9 | A25-H-B9 | A26-H-B9 | A27-H-B9 | A28-H-B9 |
| A22-H-B10 | A23-H-B10 | A24-H-B10 | A25-H-B10 | A26-H-B10 | A27-H-B10 | A28-H-810 |
| A22-H-B11 | A23-H-B11 | A24-H-B11 | A25-H-B11 | A26-H-B11 | A27-H-B11 | A28-H-B11 |
| A22-H-B12 | A23-H-B12 | A24-H-B12 | A25-H-B12 | A26-H-B12 | A27-H-B12 | A28-H-B12 |
| A22-H-B13 | A23-H-B13 | A24-H-B13 | A25-H-B13 | A26-H-B13 | A27-H-B13 | A28-H-B13 |
| A22-H-B14 | A23-H-B14 | A24-H-B14 | A25-H-814 | A26-H-B14 | A27-H-B14 | A28-H-B14 |
| A22-H-B15 | A23-H-B15 | A24-H-B15 | A25-H-B15 | A26-H-B15 | A27-H-B15 | A28-H-B15 |
| A29-H-B1 | A30-H-B1 | A31-H-B1 | A32-H-B1 | A33-H-B1 | A34-H-B1 | A35-H-B1 |
| A29-H-B2 | A30-H-B2 | A31-H-B2 | A32-H-B2 | A33-H-B2 | A34-H-B2 | A35-H-B2 |
| A29-H-B3 | A30-H-B3 | A31-H-B3 | A32-H-B3 | A33-H-B3 | A34-H-B3 | A35-H-B3 |
| A29-H-B4 | A30-H-B4 | A31-H-B4 | A32-H-B4 | A33-H-B4 | A34-H-B4 | A35-H-B4 |
| A29-H-B5 | A30-H-B5 | A31-H-B5 | A32-H-B5 | A33-H-B5 | A34-H-B5 | A35-H-B5 |
| A29-H-B6 | A30-H-B6 | A31-H-B6 | A32-H-B6 | A33-H-B6 | A34-H-B6 | A35-H-B6 |
| A29-H-B7 | A30-H-B7 | A31-H-B7 | A32-H-B7 | A33-H-B7 | A34-H-B7 | A35-H-B7 |
| A29-H-B8 | A30-H-B8 | A31-H-B8 | A32-H-B8 | A33-H-B8 | A34-H-B8 | A35-H-B8 |
| A29-H-B9 | A30-H-B9 | A31-H-B9 | A32-H-B9 | A33-H-B9 | A34-H-B9 | A35-H-B9 |
| A29-H-B10 | A30-H-B10 | A31-H-B10 | A32-H-B10 | A33-H-B10 | A34-H-B10 | A35-H-B10 |
| A29-H-B11 | A30-H-B11 | A31-H-B11 | A32-H-B11 | A33-H-B11 | A34-H-B11 | A35-H-B11 |
| A29-H-B12 | A30-H-B12 | A31-H-B12 | A32-H-B12 | A33-H-B12 | A34-H-B12 | A35-H-B12 |
| A29-H-B13 | A30-H-B13 | A31-H-B13 | A32-H-B13 | A33-H-B13 | A34-H-B13 | A35-H-B13 |
| A29-H-B14 | A30-H-B14 | A31-H-B14 | A32-H-B14 | A33-H-B14 | A34-H-B14 | A35-H-B14 |
| A29-H-B15 | A30-H-B15 | A31-H-B15 | A32-H-B15 | A33-H-B15 | A34-H-B15 | A35-H-B15 |
| A36-H-B1 | A37-H-B1 | A38-H-B1 | A39-H-B1 | A40-H-B1 | A41-H-B1 | A42-H-B1 |
| A36-H-B2 | A37-H-B2 | A38-H-B2 | A39-H-B2 | A40-H-B2 | A41-H-B2 | A42-H-B2 |
| A36-H-B3 | A37-H-B3 | A38-H-B3 | A39-H-B3 | A40-H-B3 | A41-H-B3 | A42-H-B3 |
| A36-H-B4 | A37-H-B4 | A38-H-B4 | A39-H-B4 | A40-H-B4 | A41-H-B4 | A42-H-B4 |
| A36-H-B5 | A37-H-B5 | A38-H-B5 | A39-H-B5 | A40-H-B5 | A41-H-B5 | A42-H-B5 |
| A36-H-B6 | A37-H-B6 | A38-H-B6 | A39-H-B6 | A40-H-B6 | A41-H-B6 | A42-H-B6 |
| A36-H-B7 | A37-H-B7 | A38-H-B7 | A39-H-B7 | A40-H-B7 | A41-H-B7 | A42-H-B7 |
| A36-H-B8 | A37-H-B8 | A38-H-B8 | A39-H-B8 | A40-H-B8 | A41-H-B8 | A42-H-B8 |
| A36-H-B9 | A37-H-B9 | A38-H-B9 | A39-H-B9 | A40-H-B9 | A41-H-B9 | A42-H-B9 |
| A36-H-B10 | A37-H-B10 | A38-H-B10 | A39-H-B10 | A40-H-B10 | A41-H-B10 | A42-H-B10 |
| A36-H-B11 | A37-H-B11 | A38-H-B11 | A39-H-B11 | A40-H-B11 | A41-H-B11 | A42-H-B11 |
| A36-H-B12 | A37-H-B12 | A38-H-B12 | A39-H-B12 | A40-H-B12 | A41-H-B12 | A42-H-B12 |
| A36-H-B13 | A37-H-B13 | A38-H-B13 | A39-H-B13 | A40-H-B13 | A41-H-B13 | A42-H-B13 |
| A36-H-B14 | A37-H-B14 | A38-H-B14 | A39-H-B14 | A40-H-B14 | A41-H-B14 | A42-H-B14 |
| A36-H-B15 | A37-H-B15 | A38-H-B15 | A39-H-B15 | A40-H-B15 | A41-H-B15 | A42-H-B15 |
| A43-H-B1 | A44-H-B1 | A45-H-B1 | A46-H-B1 | A47-H-B1 | A48-H-B1 | A49-H-B1 |
| A43-H-B2 | A44-H-B2 | A45-H-B2 | A46-H-B2 | A47-H-B2 | A48-H-B2 | A49-H-B2 |
| A43-H-B3 | A44-H-B3 | A45-H-B3 | A46-H-B3 | A4T-H-B3 | A48-H-B3 | A49-H-B3 |
| A43-H-B4 | A44-H-B4 | A45-H-B4 | A46-H-B4 | A47-H-B4 | A48-H-B4 | A49-H-B4 |
| A43-H-B5 | A44-H-B5 | A45-H-B5 | A46-H-B5 | A47-H-B5 | A48-H-B5 | A49-H-B5 |
| A43-H-B6 | A44-H-B6 | A45-H-B6 | A46-H-B6 | A47-H-B6 | A48-H-B6 | A49-H-B6 |
| A43-H-B7 | A44-H-B7 | A45-H-B7 | A46-H-B7 | A47-H-B7 | A48-H-B7 | A49-H-B7 |
| A43-H-B8 | A44-H-B8 | A45-H-B8 | A46-H-B8 | A47-H-B8 | A48-H-B8 | A49-H-B8 |
| A43-H-B9 | A44-H-B9 | A45-H-B9 | A46-H-B9 | A47-H-B9 | A48-H-B9 | A49-H-B9 |
| A43-H-B10 | A44-H-B10 | A45-H-B10 | A46-H-B10 | A47-H-B10 | A48-H-B10 | A49-H-B10 |
| A43-H-B11 | A44-H-B11 | A45-H-811 | A46-H-B11 | A47-H-B11 | A48-H-B11 | A49-H-B11 |
| A43-H-B12 | A44-H-B12 | A45-H-B12 | A46-H-B12 | A47-H-B12 | A48-H-B12 | A49-H-B12 |
| A43-H-B13 | A44-H-B13 | A45-H-B13 | A46-H-B13 | A47-H-B13 | A48-H-B13 | A49-H-B13 |
| A43-H-B14 | A44-H-B14 | A45-H-814 | A46-H-B14 | A47-H-B14 | A48-H-B14 | A49-H-B14 |
| A43-H-B15 | A44-H-B15 | A45-H-B15 | A46-H-B15 | A47-H-B15 | A48-H-B15 | A49-H-B15 |
| A50-H-B1 | A51-H-B1 | A52-H-B1 | A53-H-B1 | A54-H-B1 | A55-H-B1 | A56-H-B1 |
| A50-H-B2 | A51-H-B2 | A52-H-B2 | A53-H-B2 | A54-H-B2 | A55-H-B2 | A56-H-B2 |
| A50-H-B3 | A51-H-B3 | A52-H-B3 | A53-H-B3 | A54-H-B3 | A55-H-B3 | A56-H-B3 |
| A50-H-B4 | A51-H-B4 | A52-H-B4 | A53-H-B4 | A54-H-B4 | A55-H-B4 | A56-H-B4 |
| A50-H-B5 | A51-H-B5 | A52-H-B5 | A53-H-B5 | A54-H-B5 | A55-H-B5 | A56-H-B5 |
| A50-H-B6 | A51-H-B6 | A52-H-B6 | A53-H-B6 | A54-H-B6 | A55-H-B6 | A56-H-B6 |
| A50-H-B7 | A51-H-B7 | A52-H-B7 | A53-H-B7 | A54-H-B7 | A55-H-B7 | A56-H-B7 |
| A50-H-B8 | A51-H-B8 | A52-H-B8 | A53-H-B8 | A54-H-B8 | A55-H-B8 | A56-H-B8 |
| A50-H-B9 | A51-H-B9 | A52-H-B9 | A53-H-B9 | A54-H-B9 | A55-H-B9 | A56-H-B9 |
| A50-H-B10 | A51-H-B10 | A52-H-B10 | A53-H-B10 | A54-H-B10 | A55-H-B10 | A56-H-B10 |
| A50-H-B11 | A51-H-B11 | A52-H-B11 | A53-H-B11 | A54-H-B11 | A55-H-B11 | A56-H-B11 |
| A50-H-B12 | A51-H-B12 | A52-H-B12 | A53-H-B12 | A54-H-B12 | A55-H-B12 | A56-H-B12 |
| A50-H-B13 | A51-H-B13 | A52-H-B13 | A53-H-B13 | A54-H-B13 | A55-H-B13 | A56-H-B13 |
| A50-H-B14 | A51-H-B14 | A52-H-B14 | A53-H-B14 | A54-H-B14 | A55-H-B14 | A56-H-B14 |
| A50-H-B15 | A51-H-B15 | A52-H-B15 | A53-H-B15 | A54-H-B15 | A55-H-B15 | A56-H-B15 |
| A57-H-B1 | A58-H-B1 | A59-H-B1 | A60-H-B1 | A61-H-B1 | A62-H-B1 | A63-H-B1 |
| A57-H-B2 | A58-H-B2 | A59-H-B2 | A60-H-B2 | A61-H-B2 | A62-H-B2 | A63-H-B2 |
| A57-H-B3 | A58-H-B3 | A59-H-B3 | A60-H-B3 | A61-H-B3 | A62-H-B3 | A63-H-B3 |
| A57-H-B4 | A58-H-B4 | A59-H-B4 | A60-H-B4 | A61-H-B4 | A62-H-B4 | A63-H-B4 |
| A57-H-B5 | A58-H-B5 | A59-H-B5 | A60-H-B5 | A61-H-B5 | A62-H-B5 | A63-H-B5 |
| A57-H-B6 | A58-H-B6 | A59-H-B6 | A60-H-B6 | A61-H-B6 | A62-H-B6 | A63-H-B6 |
| A57-H-B7 | A58-H-B7 | A59-H-B7 | A60-H-B7 | A61-H-B7 | A62-H-B7 | A63-H-B7 |
| A57-H-B8 | A58-H-B8 | A69-H-B8 | A60-H-B8 | A61-H-B8 | A62-H-B8 | A63-H-B8 |
| A57-H-B9 | A58-H-B9 | A59-H-B9 | A60-H-B9 | A61-H-B9 | A62-H-B9 | A63-H-B9 |
| A57-H-B10 | A58-H-B10 | A59-H-B10 | A60-H-B10 | A61-H-B10 | A62-H-B10 | A63-H-B10 |
| A57-H-B11 | A58-H-B11 | A59-H-B11 | A60-H-B11 | A61-H-B11 | A62-H-B11 | A63-H-811 |
| A57-H-B12 | A58-H-B12 | A59-H-B12 | A60-H-B12 | A61-H-B12 | A62-H-B12 | A63-H-B12 |
| A57-H-B13 | A58-H-B13 | A59-H-B13 | A60-H-B13 | A61-H-B13 | A62-H-B13 | A63-H-B13 |
| A57-H-B14 | A58-H-B14 | A59-H-B14 | A60-H-B14 | A61-H-B14 | A62-H-B14 | A63-H-B14 |
| A57-H-B15 | A58-H-B15 | A59-H-B15 | A60-H-B15 | A61-H-B15 | A62-H-B15 | A63-H-B15 |
| A64-H-B1 | A65-H-B1 | A66-H-B1 | A67-H-B1 | A68-H-B1 | A69-H-B1 | A70-H-B1 |
| A64-H-B2 | A65-H-B2 | A66-H-B2 | A67-H-B2 | A68-H-B2 | A69-H-B2 | A70-H-B2 |
| A64-H-B3 | A65-H-B3 | A66-H-B3 | A67-H-B3 | A68-H-B3 | A69-H-B3 | A70-H-B3 |
| A64-H-B4 | A65-H-B4 | A66-H-B4 | A67-H-B4 | A68-H-B4 | A69-H-B4 | A70-H-B4 |
| A64-H-B5 | A65-H-B5 | A66-H-B5 | A67-H-B5 | A68-H-B5 | A69-H-B5 | A70-H-B5 |
| A64-H-B6 | A65-H-B6 | A66-H-B6 | A67-H-B6 | A68-H-B6 | A69-H-B6 | A70-H-B6 |
| A64-H-B7 | A65-H-B7 | A66-H-B7 | A67-H-B7 | A68-H-B7 | A69-H-B7 | A70-H-B7 |
| A64-H-B8 | A65-H-B8 | A66-H-B8 | A67-H-B8 | A68-H-B8 | A69-H-B8 | A70-H-B8 |
| A64-H-B9 | A65-H-B9 | A66-H-B9 | A67-H-B9 | A68-H-B9 | A69-H-B9 | A70-H-B9 |
| A64-H-B10 | A65-H-B10 | A66-H-B10 | A67-H-810 | A68-H-B10 | A69-H-B10 | A70-H-B10 |
| A64-H-B11 | A65-H-B11 | A66-H-B11 | A67-H-B11 | A68-H-B11 | A69-H-B11 | A70-H-B11 |
| A64-H-B12 | A65-H-B12 | A66-H-B12 | A67-H-B12 | A68-H-B12 | A69-H-B12 | A70-H-B12 |
| A64-H-B13 | A65-H-B13 | A66-H-B13 | A67-H-B13 | A68-H-B13 | A69-H-B13 | A70-H-B13 |
| A64-H-B14 | A65-H-B14 | A66-H-B14 | A67-H-B14 | A68-H-B14 | A69-H-B14 | A70-H-B14 |
| A64-H-B15 | A65-H-B15 | A66-H-B15 | A67-H-B15 | A68-H-B15 | A69-H-B15 | A70-H-B15 |

### Exemple 1 : 2-phényléthylamide de l'acide 3-(5,6-diméthyl-1H-benzoimidazol-2-yl)-2,4,5,7-tétrahydro-pyrazolo[3,4-c]pyridine-6-carboxylique

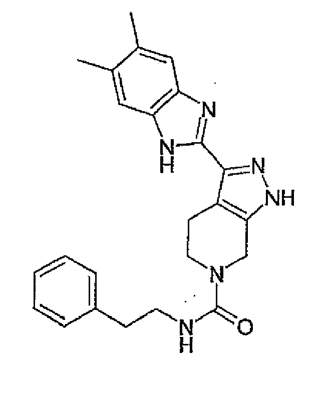

Le 2-phényléthylamide de l'acide 3-(5,6-diméthyl-1H-benzoimidazol-2-yl)-2,4,5,7-tétrahydro-pyrazolo[3,4-c]pyridine-6-carboxylique peut être préparé de la façon suivante :
10 mg de 3-(5,6-diméthyl-1H-benzimidazol-2-yl)-4,5,6,7-tétrahydro-2H-pyrazolo [3,4-c]pyridine sont mis en suspension dans 0.3 mL de tétrahydrofurane. 7.8 µL de 2-phényléthylisocyanate sont additionnés et le mélange réactionnel est agité à température ambiante pendant 20 heures puis concentré sous pression réduite.
Le résidu d'évaporation est purifié par LC/MS (Méthode B). Après purification par LC/MS, les fractions contenant le 2-phényléthylamide de l'acide3-(5,6-diméthyl-1H-benzoimidazol-2-yl)-2,4,5,7-tétrahydro-pyrazolo[3,4-c]pyridine-6-carboxylique sont réunies et déposées sur phase SCX (500 mg de phase CUBCX1-HL). La phase SCX est ensuite lavée avec du méthanol puis extraite avec une solution d'ammoniac 2M dans le méthanol. La solution d'extraction obtenue est alors concentrée sous pression réduite. On obtient ainsi 1.2 mg de 2-phényléthylamide de l'acide3-(5,6-diméthyl-1H-benzoimidazol-2-yl)-2,4,5,7-tétrahydro-pyrazolo[3,4-c]pyridine-6-carboxylique sous forme de poudre blanche dont les caractéristiques sont les suivantes :

LC/MS (Méthode A) : ion moléculaire détecté : 415.29 ; temps de rétention = 3.48 minutes

### Exemple 2 : 3-(5,6-Diméthyl-1H-benzoimidazol-2-yl)-6-méthanesulfonyl-4,5,6,7-tétrahydro-2H-pyrazolo[3,4-c]pyridine

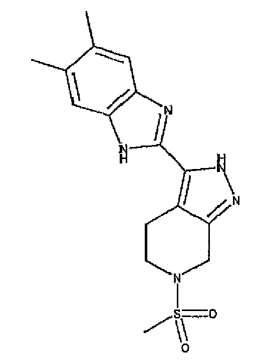

La 3-(5,6-diméthyl-1H-benzolmidazol-2-yl)-6-méthanesulfonyl-4,5,6,7-tétrahydro-2H-pyrazolo[3,4-c]pyridine peut être préparée de la façon suivante:
10 mg de 3-(5,6-diméthyl-1H-benzimidazol-2-yl)-4,5,6,7-tétrahydro-2H-pyrazolo[3,4-c]pyridine sont mis en suspension dans 0.3 mL de dichlorométhane. 15.8 µL de triéthylamine sont additionnés ainsi que 4.5 µL de chlorure de méthanesulfonyle. Le mélange réactionnel est agité à température ambiante pendant 20 heures puis concentré sous pression réduite.
Le résidu d'évaporation est purifié par LC/MS (Méthode B). Après purification par LC/MS, les fractions contenant la 3-(5,6-diméthyl-1H-bonzolmidazol-2-yl)-6-méthanesulfonyl-4,5,6,7-tétrahydro-2H-pyrazolo[3,4-c]pyridine sont réunies et déposées sur phase SCX (500 mg de phase CUBCX1-HL). La phase SCX est ensuite lavée avec du méthanol puis extraite avec une solution d'ammoniac 2M dans le méthanol. La solution d'extraction obtenue est alors concentrée sous pression réduite. On obtient ainsi 4.2 mg de 3-(5,6-diméthyl-1H-benzoimidazol-2-yl)-6-méthanesulfonyl-4,5,6,7-tétrahydro-2H-pyrazolo [3,4-c]pyridine sous forme de poudre blanche dont les caractéristiques sont les suivantes :

LC/MS (Méthode A) : ion moléculaire détecté : 346.30 ; temps de rétention = 3.08 minutes

R.M.N. ¹H (300 MHz, (CD₃)₂SO, δ en ppm) : 2,32 (s large : 6H) ; 3,02 (s : 3H) ; 3,03 (mt : 2H) ; 3,52 (t large, J = 5 Hz : 2H) ; 4,45 (s large : 2H) ; 7,24 (s large : 1H) ; 7,42 (s large : 1H) ; 12,45 (mf : 1H) ; 13,07 (mf : 1H).

### Exempte 3 : [3-(5,6-Diméthyl-1H-benzolmidazol-2-yl)-2,4,5,7-tétrahydro-pyrazolo[3,4-c]pyridin-6-yl]-3-pyridinyl-méthanone

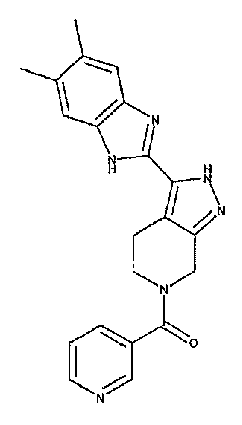

La [3-(5,6-diméthyl-1H-benzoimidazol-2-yl)-2,4,5,7-tétrahydro-pyrazolo[3,4-c]pyridin-6-yl]-3-pyridinyl-méthanone peut être préparée de la façon suivante : 10 mg de 3-(5,6-diméthyl-1H-benzimidazol-2-yl)-4,5,6,7-tétrahydro-2H-pyrazolo[3,4-c]pyridine sont mis en suspension dans 0.3 mL de DMF. 6.9 mg d'acide nicotinique sont additionnés, suivis de 7.6 mg de HOBT et 8.7 µL de diisopropylcarbodiimide. Le mélange réactionnel est agité à température ambiante pendant 20 heures puis concentré sous pression réduite.
Le résidu d'évaporation est purifié par LC/MS (Méthode B). Après purification par LC/MS, les fractions contenant la [3-(5,6-diméthyl-1H-benzoimidazol-2-yl)-2,4,5,7-tétrahydro-pyrazolo[3,4-c]pyridin-6-yl]-3-pyridinyl-méthanone sont réunies et déposées sur phase SCX (500 mg de phase CUBCX1-HL). La phase SCX est ensuite lavée avec du méthanol puis extraite avec une solution d'ammoniac 2M dans le méthanol. La solution d'extraction obtenue est alors concentrée sous pression réduite. On obtient ainsi 5.3 mg de [3-(5,6-diméthyl-1H-benzoimidazol-2-yl)-2,4,5,7-tétrahydro-pyrazolo[3,4-c]pyridin-6-yl]-3-pyridinyl-méthanone sous forme de poudre blanche dont les caractéristiques sont les suivantes :

LC/MS (Méthode A) : ion moléculaire détecté : 373.31 ; temps de rétention = 2.87 minutes

R.M.N. ¹H (400 MHz, (CD₃)₂SO, à une température de 373K, δ en ppm): 2,35 (s : 6H) ; de 2,90 à 3,10 (mt : 2H) ; 3,76 (mf : 2H) ; 4,76 (s large : 2H) ; 7,27 (mf : 1H) ; 7,40 (mf : 1H) ; 7,51 (dd, J = 8 et 5 Hz : 1H) ; 7,90 (d large, J = 8 Hz : 1H) ; 8,70 (mt : 2H) ; de 11,80 à 12,20 (mf étalé: 1H) ; de 12,50 à 13,00 (mf étalé : 1H).

### Exemple 4 : 6-(3-Chloro-benzyl)-3-(5,6-diméthyl-1H-benzoimidazol-2-yl)-4,5,6,7-tétrahydro-2H-pyrazolo[3,4-c]pyridine

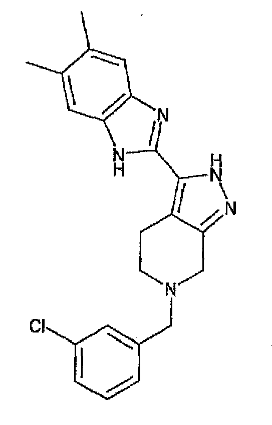

La 6-(3-chloro-benzyl)-3-(5,6-diméthyl-1H-benzoimidazol-2-yl)-4,5,6,7-tétrahydro-2H-pyrazolo[3,4-c]pyridine peut être préparée de la façon suivante :
10 mg de 3-(5,6-diméthyl-1H-benzimidazol-2-yl)-4,5,6,7-tétrahydro-2H-pyrazolo[3,4-c]pyridine sont mis en suspension dans 0.3 mL de méthanol. 12.7 µL de 3-chlorobenzaldéhyde sont additionnés, suivis de 4.7 mg de NaBH₃CN. Le mélange réactionnel est agité à température ambiante pendant 20 heures puis concentré sous pression réduite.
Le résidu d'évaporation est purifié par LC/MS (Méthode B). Après purification par LC/MS, les fractions contenant la 6-(3-chloro-benzyl)-3-(5,6-diméthyl-1H-benzoimidazol-2-yl)-4,5,6,7-tétrahydro-2H-pyrazolo[3,4-c]pyridine sont réunies et déposées sur phase SCX (500 mg de phase CUBCX1-HL). La phase SCX est ensuite lavée avec du méthanol puis extraite avec une solution d'ammoniac 2M dans le méthanol. La solution d'extraction obtenue est alors concentrée sous pression réduite. On obtient ainsi 4 mg de 6-(3-chloro-benzyl)-3-(5,6-diméthyl-1H-benzoimidazol-2-yl)-4,5,6,7-tétrahydro-2H-pyrazolo[3,4-c]pyridine sous forme de poudre blanche dont les caractéristiques sont les suivantes :

LC/MS (Méthode A) : ion moléculaire détecté : 392.26 ; temps de rétention = 3.18 minutes

R.M.N. ¹H (400 MHz, (CD₃)₂SO, à une température de 373K, δ en ppm) : 2,35 et 2,36 (2 s : 6H en totalité) ; 2,83 (t, J = 5,5 Hz : 2H) ; de 2,90 à 3,00 (mt : 2H) ; 3,62 (s large : 2H) ; 3,78 (s : 2H) ; de 7,25 à 7,50 (mt : 6H) ; 11,91 (mf : 1H) ; de 12,30 à 12.60 (mf étalé : 1H).

### Exemple 5 : [3-(1H-benzoimidazol-2-yl)-2,4,5,7-tétrahydro-pyrazolo[3,4-c] pyndin-6-yl]-3-pyridinyl-méthanone

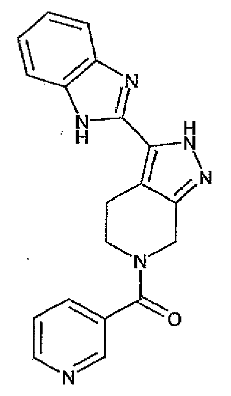

La [3-(1H-benzoimidazol-2-yl)-2,4,5,7-tétrahydro-pyrazolo[3,4-c]pyridin-6-yl]-3-pyridinyl-méthanone peut être préparée de la façon suivante :
15 mg de chlorhydrate de la 3-(1H-benzimidazol-2-yl)-4,5,6,7-tétrahydro-2H-pyrazolo[3,4-c]pyridine sont mis en suspension dans 0.5 mL de DMF. 24.3 mg de diisopropyléthylamine sont additionnés, suivis de 12.7 mg de HOBT, 11.9 mg de diisopropylcarbodiimide et de 11.6 mg d'acide nicotinique. Le mélange réactionnel est agité à température ambiante pendant 20 heures puis concentré sous pression réduite.
Le résidu d'évaporation est purifié par LC/MS (Méthode B). Après purification par LC/MS, les fractions contenant la [3-(1H-benzoimidazol-2-yl)-2,4,5,7-tétrahydro-pyrazolo[3,4-c]pyridin-6-yl]-3-pyridinyl-méthanone sont réunies et déposées sur phase SCX (500 mg de phase CUBCX1-HL). La phase SCX est ensuite lavée avec du méthanol puis extraite avec une solution d'ammoniac 2M dans le méthanol. La solution d'extraction obtenue est alors concentrée sous pression réduite. On obtient ainsi 7.7 mg de [3-(1H-benzoimidazol-2-yl)-2,4,5,7-tétrahydro-pyrazolo[3,4-c]pyridin-6-yl]-3-pyridinyl-méthanone sous forme de poudre blanche dont les caractéristiques sont les suivantes :

LC/MS (Méthode A) : ion moléculaire détecté : 345.22 ; temps de rétention = 1.95 minutes

### Exemple 6 : 6-(3-Chloro-benzyl)-3-(1H-benzolmidazol-2-yl)-4,5,6,7-tétrahydro-2H-pyrazolo[3,4-c]pyridine

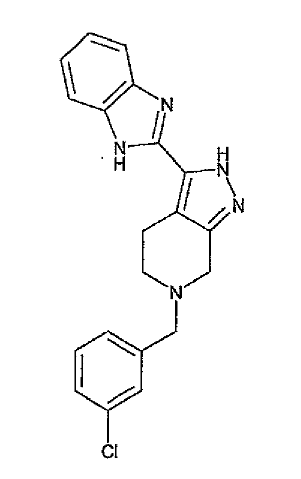

La 6-(3-chloro-benzyl)-3-(1H-benzoimidazol-2-yl)-4,5,6,7-tétrahydro-2H-pyrazolo[3,4-c]pyridine peut être préparée de la façon suivante :
15 mg de chlorhydrate de la 3-(1H-benzimidazal-2-yl)-4,5,6,7-tétrahydro-2H-pyrazolo[3,4-c]pyridine sont mis en suspension dans 0.5 mL de méthanol. 26.5 mg de 3-ehlorobenzaldéhyde sont additionnés, suivis de 7.9 mg de NaBH₃CN, Le mélange réactionnel est agité à température ambiante pendant 20 heures puis concentré sous pression réduite.
Le résidu d'évaporation est purifié par LC/MS (Méthode B). Après purification par LC/MS, les fractions contenant la 6-(3-chloro-benzyl)-3-(1H-benzoimidazol-2-yl)-4,5,6,7-tétrahydro-2H-pyrazolo[3,4-c]pyridine sont réunies et déposées sur phase SCX (500 mg de phase CUBCX1-HL). La phase SCX est ensuite lavée avec du méthanol puis extraite avec une solution d'ammoniac 2M dans le méthanol. La solution d'extraction obtenue est alors concentrée sous pression réduite. On obtient ainsi 6.9 mg de 6-(3-chloro-benzyl)-3-(1H-benzoimidazol-2-yl)-4,5,6,7-tétrahydro-2H-pyrazolo[3,4-c]pyridine sous forme de poudre blanche dont les caractéristiques sont les suivantes :

LC/MS (Méthode A) : ion moléculaire détecté : 364.22 ; temps de rétention = 2.19 minutes

### Exemple 7 : Préparation d'une librairie d'amide

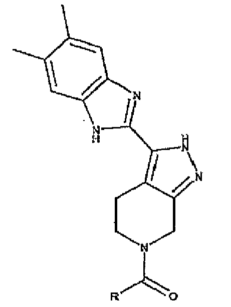

La librairie d'amides peut être préparée de la façon suivante :
Les 19 acides (tableau 4) sont pesés et placés dans 19 tubes à essais individuels.

**Tableau 4 : acides utilisés**

| Entrée | Nom | Quantité |
|---|---|---|
| 1 | ACIDE ISOBUTYRIQUE | 3.3 mg |
| 2 | ACIDE BENZOIQUE | 4.6 mg |
| 3 | ACIDE 2,3-DICHLOROBENZOIQUE | 7.1 mg |
| 4 | ACIDE PHENYLACETIQUE | 5.1 mg |
| 5 | ACIDE ACETIQUE | 2.2 mg |
| 6 | ACIDE CYCLOPROPANECARBOXYLIQUE | 3.2 mg |
| 7 | ACIDE 2-CHLOROBENZOIQUE | 5.9 mg |
| 8 | ACIDE 3-CHLOROBENZOIQUE | 5.9 mg |
| 9 | ACIDE 4-CHLOROBENZOIQUE | 5.9 mg |
| 10 | ACIDE ISOVALERIQUE | 3.8 mg |
| 11 | ACIDE HYDROCINNAMIQUE | 5.6 mg |
| 12 | ACIDE VINYLACETIQUE | 3.2 mg |
| 13 | ACIDE BUTYRIQUE | 3.3 mg |
| 14 | ACIDE 2-FUROIQUE | 4.2 mg |
| 15 | ACIDE PIVALIQUE | 3.8 mg |
| 16 | N,N-DIMETHYLGLYCINE | 3.9 mg |
| 17 | ACIDE VALERIQUE | 3.8 mg |
| 18 | ACIDE THIOPHENE-2-CARBOXYLIQUE | 4.8 mg |
| 19 | ACIDE 4-METHYLSULFONYLBENZOIQUE | 7.5 mg |

152 mg de HOBT et 142 mg de diisopropylcarbodiimide sont placés en solution dans 12 mL de DMF et la solution obtenue est distribuée dans chacun des 19 tubes à essais, à raison de 600 µL par tube.
200 mg de chlorhydrate de la 3-(5,6-diméthyl-1H-benzimidazol-2-yl)-4,5,6,7-tétrahydro-2H-pyrazolo[3,4-c]pyridine sont mis en suspension dans 4 mL de DMF en présence de 290 mg de N,N-diisopropyléthylamine et la suspension obtenue est distribuée dans chacun des 19 tubes à essais, à raison de 200 µL par tube.
Les 19 mélanges réactionnels sont agités par agitation orbitalaire à température ambiante pendant 20 heures.
Pour chaque mélange réactionnel, un prélèvement de 10 µL est effectué et dilué dans 40 µL de DMSO (Gilson Liquid Handler Quad-Z 215). Chaque échantillon en solution dans le DMSO ainsi obtenu, est analysé par LC/MS (Méthode A).

Les 19 mélanges réactionnels sont ensuite évaporés à sec et les résidus d'évaporation sont placés en solution dans 500 µL de DMSO chacun, puis les solutions obtenues sont purifiées par LC/MS (Méthode B).
Après purification par LC/MS, les fractions contenant les composés cherchés sont (éventuellement réunies) déposées sur phase SCX (500 mg de phase CUBCX1-HL). Les phases SCX sont ensuite lavées avec du méthanol puis extraites avec une solution d'ammoniac 2M dans le méthanol. Les solutions d'extraction sont collectées en tubes de verre tarés, évaporées à sec (évaporateur centrifuge Savant AES 2000 ou Genevac HT8), pesées (Mettler Toledo Automated Workstation LA200) et diluées à 10 mM dans le DMSO (Gilson Liquid Handler Quad-Z 215). Chaque solution obtenue est analysée par LC/MS (Méthode A).

Les composés suivants (Tableau 5), ont été isolés et caractérisés par leur temps de rétention et pic moléculaire en Spectrométrie de Masse (Méthode A).

**Tableau 5 : librairie d'amides obtenue**

| Entrée | Nom | Quantité de produit obtenue | Temps de rétention (minutes) | Ion moléculaire détecté |
|---|---|---|---|---|
| 1 | 1-[3-(5,6-Diméthyl-1H-benzoimidazol-2-yl)-2,4,5,7-tétrahydro-pyrazolo[3,4-c]pyridin-6-yl]-2-méthyl-propan-1-one | 5.8 mg | 3.08 | 338.23 |
| 2 | [3-(5,6-Diméthyl-1H-benzoimidazol-2-yl)-2,4,5,7-tétrahydro-pyrazolo[3,4-c]pyridin-6-yl]-phényl-méthanone | 6.8 mg | 2.68 | 372.21 |
| 3 | (2,3-Dichloro-phényl)-[3-(5,6-diméthyl-1H-benzoimidazol-2-yl)-2,4,5,7-tétrahydro-pyrazolo[3,4-c]pyridin-6-yl]-méthanone | 12 mg | 3.05 | 440.13 |
| 4 | 1-[3-(5,6-Diméthyl-1H-benzoimidazol-2-yl)-2,4,5,7-tétrahydro-pyrazolo[3,4-c]pyridin-6-yl]-2-phényl-éthanone | 7.9 mg | 2.99 | 386.23 |
| 5 | 1-[3-(5,6-Diméthyl-1H-benzoimidazol-2-yl)-2,4,5,7-tétrahydro-pyrazolo[3,4-c]pyndin-6-yl]-éthanone | 2.7 mg | 2.4 | 310.19 |
| 6 | Cyclopropyl-[3-(5,6-diméthyl-1H-benzoimidazol-2-yl)-2,4,5,7-tétrahydro-pyrazolo[3,4-c]pyridin-6-yl]-méthanone | 3.4 mg | 2.57 | 336.21 |
| 7 | (2-Chloro-phényl)-[3-(5,6-diméthyl-1H-benzoimidazol-2-yl)-2,4,5,7-tétrahydro-pyrazolo[3,4-c]pyridin-6-yl]-méthanone | 11.2 mg | 2.97 | 406.18 |
| 8 | (3-Chloro-phényl)-[3-(5,6-diméthyl-1H-benzoimidazol-2-yl)-2,4,5,7-tétrahydro-pyrazolo[3,4-c]pyridin-6-yl]-méthanone | 12.1 mg | 3.31 | 406.16 |
| 9 | (4-Chloro-phényl)-[3-(5,6-diméthyl-1H-benzoimidazol-2-yl)-2,4,5,7-tétrahydro-pyrazolo[3,4-c]pyridin-6-yl]-méthanone | 11.5 mg | 3.51 | 406.17 |
| 10 | 1-[3-(5,6-Diméthyl-1H-benzoimidazol-2-yl)-2,4,5,7-tétrahydro-pyrazolo[3,4-c]pyridin-6-yl]-3-méthyl-butan-1-one | 4.8 mg | 2.72 | 352.24 |
| 11 | 1-[3-(5,6-Diméthyl-1H-benzolmidazol-2-yl)-2,4,5,7-tétrahydro-pyrazolo[3,4-c]pyndin-6-yl]-3-phényl-propan-1-one | 11.9 mg | 2.95 | 400.24 |
| 12 | 1-[3-(5,6-Diméthyl-1H-benzoimidazol-2-yl)-2,4,5,7-tétrahydro-pyrazolo[3,4-c]pyridin-6-yl]-but-3-en-1-one | 10.1 mg | 2.72 | 336.22 |
| 13 | 1-[3-(5,6-Diméthyl-1H-benzoimidazol-2-yl)-2,4,5,7-tétrahydro-pyrazolo[3,4-c]pyridin-6-yl]-butan-1-one | 7 mg | 2.66 | 338.23 |
| 14 | [3-(5,6-Diméthyl-1H-benzoimidazol-2-yl)-2,4,5,7-tétrahydro-pyrazolo[3,4-c]pyridin-6-yl]-furan-2-yl-méthanone | 9.5 mg | 2.67 | 362.19 |
| 15 | 1-[3-(5,6-Diméthyl-1H-benzoimidazol-2-yl)-2,4,5,7-tétrahydro-pyrazolo[3,4-c]pyridin-6-yl]-2,2-diméthyl-propan-1-one | 9.3 mg | 2.8 | 352.24 |
| 16 | 2-Diméthylamino-1-[3-(5, 6-diméthyl-1H-benzoimidazol-2-yl)-2,4,5,7-tétrahydro-pyrazolo[3,4-c]pyridin-6-yl]-éthanone | 4.7 mg | 2.55 | 353.23 |
| 17 | 1-[3-(5,6-Diméthyl-1H-benzoimidazol-2-yl)-2,4,5,7-tétrahydro-pyrazolo[3,4-c]pyridin-6-yl]-pentan-1-one | 5.4 mg | 2.78 | 352.24 |
| 18 | [3-(5,6-Diméthyl-1H-benzoimidazol-2-yl)-2,4,5,7-tétrahydro-pyrazolo[3,4-c]pyridin-6-yl]-thiophen-2-yl-méthanone | 7.2 mg | 2.75 | 378.17 |
| 19 | [3-(5,6-Diméthyl-1H-benzoimidazol-2-yl)-2,4,5,7-tétrahydro-pyrazolo[3,4-c]pyridin-6-yl]-(4-méthanesulfonyl-phényl)-méthanone | 14.3 mg | 2.79 | 450.19 |

### Exemple 8 : Préparation d'une librairie de sulfonamides

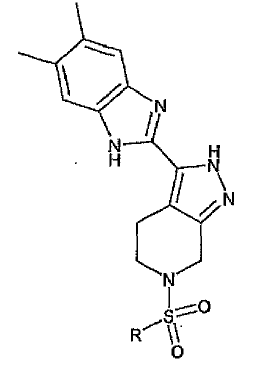

La librairie de sulfonamides peut être préparée de la façon suivante : 190 mg de chlorhydrate de la 3-(5,6-diméthyl-1H-benzimidazol-2-yl)-4,5,6,7-tétrahydro-2H-pyrazolo[3,4-c]pyridine sont mis en suspension dans 2 mL de dichlorométhane en présence de 150 µL de triéthylamine et la suspension obtenue est distribuée dans 17 tubes à essais, à raison de 500 µL par tube.
Les 17 chlorures de sulfonyles (tableau 6) sont pesés et additionnés dans chacun des 17 tubes à essais.

**Tableau 6 : Chlorures de sulfonyle utilisés**

| Entrée | Nom | Quantité |
|---|---|---|
| 1 | CHLORURE DE BENZENESULFONYLE | 9.9 mg |
| 2 | CHLORURE DE ALPHA-TOLUENESULFONYLE | 10.7 mg |
| 3 | CHLORURE DE 2,3-DICHLOROBENZENESULFONYLE | 13.8 mg |
| 4 | CHLORURE DE 4-CHLOROBENZENESULFONYLE | 11.9 mg |
| 5 | CHLORURE DE 2,2,2-TRIFLUOROETHANESULFONYLE | 10.2 mg |
| 6 | CHLORURE DE ETHANESULFONYLE | 7.2 mg |
| 7 | CHLORURE DE 1-PROPANESULFONYLE | 8 mg |
| 8 | CHLORURE DE 1-BUTANESULFONYLE | 8.8 mg |
| 9 | CHLORURE DE 2-CHLOROBENZENESULFONYLE | 11.9 mg |
| 10 | CHLORURE DE 3-CHLOROBENZENESULFONYLE | 11.9 mg |
| 11 | CHLORURE DE [(4-FLUOROPHENYL)METHYL]SULFONYLE | 11.7 mg |
| 12 | CHLORURE DE 4-METHOXYBENZENESULFONYLE | 11.6 mg |
| 13 | CHLORURE DE P-TOLUENESULFONYLE | 10.7 mg |
| 14 | CHLORURE DE O-TOLUENESULFONYLE | 10.7 mg |
| 15 | CHLORURE DE 3-METHYLBENZENESULFONYLE | 10.7 mg |
| 16 | CHLORURE DE 3-METHOXYBENZENESULPHONYLE | 11.6 mg |
| 17 | CHLORURE DE 2-METHOXY-4-METHYLBENZENESULPHONYLE | 12.4 mg |

Les 17 mélanges réactionnels sont agités par agitation orbitalaire à température ambiante pendant 20h.
Pour chaque mélange réactionnel, un prélèvement de 10 µL est effectué et dilué dans 40 µL de DMSO (Gilson Liquid Handler Quad-Z 215). Chaque échantillon en solution dans le DMSO ainsi obtenu, est analysé par LC/MS (Méthode A).

Les 17 mélanges réactionnels sont ensuite évaporés à sec et les résidus d'évaporation sont placés en solution dans 1 mL de DMSO chacun en présence d'une goutte d'une solution aqueuse d'acide chlorhydrique 5N et les solutions obtenues sont purifiées par LC/MS (Méthode B). Après purification par LC/MS, les fractions contenant les composés cherchés sont (éventuellement réunies) déposées sur phase SCX (500 mg de phase CUBCX1-HL). Les phases SCX sont ensuite lavées avec du méthanol puis extraites avec une solution d'ammoniac 2M dans le méthanol. Les solutions d'extraction sont collectées en tubes de verre tarés, évaporées à sec (évaporateur centrifuge Savant AES 2000 ou Genevac HT8), pesées (Mettler Toledo Automated Workstation LA200) et diluées à 10 mM dans le DMSO (Gilson Liquid Handler Quad-Z 215). Chaque solution obtenue est analysée par LC/MS (Méthode A).

Les composés suivants (Tableau 7), ont été isolés et caractérisés par leur temps de rétention et pic moléculaire en Spectrométrie de Masse (Méthode A).

**Tableau 7 : librairie de sulfonamides obtenue**

| Entrée | Nom | Quantité de produit obtenue | Temps de rétention (minutes) | Ion moléculaire détecté |
|---|---|---|---|---|
| 1 | 6-Benzenesulfonyl-3-(5,6-diméthyl-1H-benzoimidazol-2-yl)-4,5,6,7-tétrahydro-2H-pyrazolo[3,4-c]pyridine | 1.4 mg | 3.41 | 408.18 |
| 2 | 3-(5,6-Diméthyl-1H-benzoimidazol-2-yl)-6-phénylméthanesulfonyl-4,5,6,7-tétrahydro-2H-pyrazolo[3,4-c]pyridine | 0.7 mg | 3.51 | 422.2 |
| 3 | 6-(2,3-Dichloro-benzenesulfonyl)-3-(5,6-diméthyl-1H-benzoimidazol-2-yl)-4,5,6,7-tétrahydro-2H-pyrazolo[3,4-c]pyridine | 6.4 mg | 3.25 | 476.1 |
| 4 | 6-(4-Chloro-benzenesulfonyl)-3-(5,6-diméthyl-1H-benzoimidazol-2-yl)-4,5,6,7-tétrahydro-2H-pyrazolo[3,4-c]pyridine | 5.9 mg | 3.19 | 442.12 |
| 5 | 3-(5,6-Diméthyl-1H-benzoimidazol-2-yl)-6-(2,2,2-trifluoro-éthanesulfonyl)-4,5,6,7-tétrahydro-2H-pyrazolo[3,4-c]pyridine | 1.7 mg | 3.06 | 414.14 |
| 6 | 3-(5,6-Diméthyl-1H-benzolmidazol-2-yl)-6-éthanesulfonyl-4,5,6,7-tétrahydro-2H-pyrazolo[3,4-c]pyridine | 5.2 mg | 2.63 | 360.17 |
| 7 | 3-(5,6-Diméthyl-1H-benzoimidazol-2-yl)-6-(propane-1-sulfonyl)-4,5,6,7-tétrahydro-2H-pyrazolo[3,4-c]pyridine | 4.3 mg | 2.8 | 374.19 |
| 8 | 6-(Butane-1-sulfonyl)-3-(5,6-diméthyl-1H-benzoimidazol-2-yl)-4,5,6,7-tétrahydro-2H-pyrazolo[3,4-c]pyridine | 5.6 mg | 2.94 | 388.2 |
| 9 | 6-(2-Chloro-benzenesulfonyl)-3-(5,6-diméthyl-1H-benzolmidazol-2-yl)-4,5,6,7-tétrahydro-2H-pyrazolo[3,4-c]pyridine | 5.6 mg | 3.38 | 442.13 |
| 10 | 6-(3-Chloro-benzenesulfonyl)-3-(5,6-diméthyl-1H-banzoimidazol-2-yl)-4,5,6,7-tétrahydro-2H-pyrazolo[3,4-c]pyridine | 6.9 mg | 3.71 | 442.13 |
| 11 | 3-(5,6-Diméthyl-1H-benzoimidazol-2-yl)-6-(4-fluoro-phénylméthanesulfonyl)-4,5,6,7-tétrahydro-2H-pyrazolo[3,4-c]pyridine | 0.7 mg | 3.05 | 440.18 |
| 12 | 3-(5,6-Diméthyl-1H-benzoimidazol-2-yl)-6-(4-methoxy-benzenesulfonyl)-4,5,6,7-tétrahydro-2H-pyrazolo[3,4-c]pyridine | 7.5 mg | 2.99 | 438.19 |
| 13 | 3-(5,6-Diméthyl-1H-benzoimidazol-2-yl)-6-(toluene-4-sulfonyl)-4,5,6,7-tétrahydro-2H-pyrazolo[3,4-c]pyrldine | 7.3 mg | 3.22 | 422.2 |
| 14 | 3-(5,6-Diméthyl-1H-benzoimidazol-2-yl)-6-(toluene-2-sulfonyl)-4,5,6,7-tétrahydro-2H-pyrazofo[3,4-c]pyirdine | 4.8 mg | 3.16 | 422.19 |
| 15 | 3-(5,6-Diméthyl-1H-benzoimidazol-2-yl)-6-(toluene-3-sulfonyl)-4,5,6,7-tétrahydro-2H-pyrazolo[3,4-c]pyridine | 5.1 mg | 3.13 | 422.19 |
| 16 | 3-(5,6-Diméthyl-1H-benzoimidazol-2-yl)-6-(3-methoxy-benzenesulfonyl)-4,5,6,7-tétrahydro-2H-pyrazolo[3,4-c]pyridine | 6.9 mg | 3.07 | 438.18 |
| 17 | 3-(5,6-Diméthyl-1H-bonzoimidazol-2-yl)-6-(2-methoxy-4-méthyl-benzenesulfonyl)-4,5,6,7-tétrahydro-2H-pyrazolo[3,4-c]pyridine | 0.8 mg | 3.34 | 452.19 |

### Exemple 9 : Préparation d'une librairie d'amines

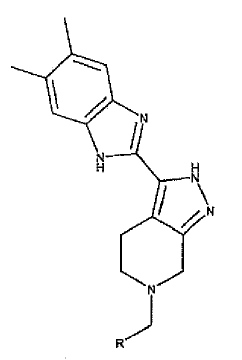

La librairie d'amines peut être préparée de la façon suivante :
180 mg de chlorhydrate de la 3-(5,6-diméthyl-1H-benzimidazol-2-yl)-4,5,6,7-tétrahydro-2H-pyrazolo[3,4-c]pyridine sont mis en suspension dans 2.7 mL de méthanol et la suspension obtenue est distribuée dans 16 tubes à essais, à raison de 150 µL par tube.

Les 16 aldéhydes (tableau 8) sont pesés et additionnés dans chacun des 16 tubes à essais.

**Tableau 8 : Aldéhydes utilisés**

| Entrée | Nom | Quantité |
|---|---|---|
| 1 | ISOBUTYRALDEHYDE | 8.1 mg |
| 2 | FORMALDEHYDE | 3.4 mg |
| 3 | BENZALDEHYDE | 11.9 mg |
| 4 | PHENYLACETALDEHYDE | 13.5 mg |
| 5 | 2,3-DICHLOROBENZALDEHYDE | 19.6 mg |
| 6 | FURFURAL | 10.8 mg |
| 7 | 4-CHLOROBENZALDEHYDE | 15.8 mg |
| 8 | 2-THIOPHENECARBOXALDEHYDE | 12.6 mg |
| 9 | NICOTINALDEHYDE | 12 mg |
| 10 | TRIMETHYLACETALDEHYDE | 9.7 mg |
| 11 | ACETALDEHYDE | 4.9 mg |
| 12 | ISOVALERALDEHYDE | 9.7 mg |
| 13 | PROPIONALDEHYDE | 6.5 mg |
| 14 | 3-PHENYLPROPIONALDEHYDE | 15.1 mg |
| 15 | BUTYRALDEHYDE | 8.1 mg |
| 16 | CYCLOPROPANECARBOXALDEHYDE | 7.9 mg |

Une solution de 85 mg de NaBH₃CN dans 2.7 mL de méthanol est alors également distribuée dans les 16 tubes à essais, à raison de 150 µL par tube. Les 16 mélanges réactionnels sont agités par agitation orbitalaire à température ambiante pendant 20h. 100 µL de méthanol sont alors ajoutés dans chacun des 16 tubes.
Pour chaque mélange réactionnel, un prélèvement de 10 µL est effectué et dilué dans 40 µL de DMSO (Gilson Liquid Handler Quad-Z 215). Chaque échantillon en solution dans le DMSO ainsi obtenu, est analysé par LC/MS (Méthode A).

Les 16 mélanges réactionnels sont ensuite évaporés à sec et les résidus d'évaporation sont placés en solution dans 500 µL de DMSO chacun, filtrés sur fritté, puis les solutions résiduelles sont purifiées par LC/MS (Méthode B). Après purification par LC/MS, les fractions contenant les composés cherchés sont (éventuellement réunies) déposées sur phase SCX (500 mg de phase CUBCX1-HL). Les phases SCX sont ensuite lavées avec du méthanol puis extraites avec une solution d'ammoniac 2M dans le méthanol. Les solutions d'extraction sont collectées en tubes de verre tarés, évaporées à sec (évaporateur centrifuge Savant AES 2000 ou Genevac HT8), pesées (Mettler Toledo Automated Workstation LA200) et diluées à 10 mM dans le DMSO (Gilson Liquid Handler Quad-Z 215). Chaque solution obtenue est analysée par LC/MS (Méthode A).

Les composés suivants (Tableau 9), ont été isolés et caractérisés par leur temps de rétention et pic moléculaire en Spectrométrie de Masse (Méthode A).

**Tableau 9 : librairie d'amines obtenue**

| Entrée | Nom | Quantité de produit obtenue | Temps de rétention (minutes) | Ion moléculaire détecté |
|---|---|---|---|---|
| 1 | 3-(5,6-Diméthyl-1H-benzoimidazol-2-yl)-6-isobutyl-4,5,6,7-tétrahydro-2H-pyrazolo[3,4-c]pyridine | 5.9 mg | 2.62 | 324.32 |
| 2 | 3-(5,6-Diméthyl-1H-benzoimidazol-2-yl)-6-méthyl-4,6,6,7-tétrahydro-2H-pyrazolo[3,4-c]pyridine | 3.5 mg | 2.49 | 282.29 |
| 3 | 6-Benzyl-3-(5,6-diméthyl-1H-benzoimidazol-2-yl)-4,5,6,7-tétrahydro-2H-pyrazolo[3,4-c]pyridine | 8.2 mg | 2.74 | 358.3 |
| 4 | 3-(5,6-Diméthyl-1H-benzoimidazol-2-yl)-6-phenéthyl-4,5,6,7-tétrahydro-2H-pyrazolo[3,4-c]pyridine | 6.4 mg | 2.84 | 372.32 |
| 5 | 6-(2,3-Dichloro-benzyl)-3-(5,6-diméthyl-1H-benzolmidazol-2-yl)-4,5,6,7-tétrahydro-2H-pyrazolo[3,4-c]pyridine | 8.6 mg | 2.95 | 426.23 |
| 6 | 3-(5,6-Diméthyl-1H-benzoimidazol-2-yl)-6-furan-2-ylméthyl-4,5,6,7-tétrahydro-2H-pyrazolo[3,4-c]pyridine | 5.9 mg | 2.64 | 348.27 |
| 7 | 6-(4-Chloro-benzyl)-3-(5,6-diméthyl-1H-benzoimidazol-2-yl)-4,5,6,7-tétrahydro-2H-pyrazolo[3,4-c]pyridine | 4.7 mg | 2.9 | 392.26 |
| 8 | 3-(5,6-Diméthyl-1H-benzoimidazol-2-yl)-6-thiophen-2-ylméthyl-4,5,6,7-tétrahydro-2H-pyrazolo[3,4-c]pyridine | 8.4 mg | 2.71 | 364.24 |
| 9 | 3-(5,6-Diméthyl-1H-benzoimidazol-2-yl)-6-pyridin-3-ylméthyl-4,5,6,7-tétrahydro-2H-pyrazolo[3,4-c]pyridine | 11.7 mg | 2.55 | 359.29 |
| 10 | 3-(5,6-Diméthyl-1H-benzoimidazol-2-yl)-6-(2,2-diméthyl-propyl)-4,5,6,7-tétrahydro-2H-pyrazob[3,4-c]pyridine | 3.7 mg | 2.72 | 338.32 |
| 11 | 3-(5,6-Diméthyl-1H-benzoimidazol-2-yl)-6-éthyl-4,5,6,7-tétrahydro-2H-pyrazolo[3,4-c]pyridine | 5 mg | 2.55 | 296.27 |
| 12 | 3-(5,6-Diméthyl-1H-benzoimidazal-2-yl)-6-(3-méthyl-butyl)-4,5,6,7-tétrahydro-2H-pyrazolo[3,4-c]pyridine | 5.9 mg | 2.76 | 338.3 |
| 13 | 3-(5,6-Diméthyl-1H-benzoimidazol-2-yl)-6-propyl-4,5,6,7-tétrahydro-2H-pyrazolo[3,4-c]pyridine | 6.4 mg | 2.62 | 310.29 |
| 14 | 3-(5,6-Diméthyl-1H-benzoimidazol-2-yl)-6-(3-phényl-propyl)-4,5,6,7-tétrahydro-2H-pyrazolo[3,4-c]pyridine | 4.2 mg | 2.97 | 386.31 |
| 15 | 6-Butyl-3-(5,6-diméthyl-1H-benzolmidazol-2-yl)-4,5,6,7-tétrahydro-2H-pyrazolo[3,4-c]pyridine | 4.5 mg | 2.68 | 324.28 |
| 16 | 6-Cyclopropylméthyl-3-(5,6-diméthyl-1H-benzoimidazol-2-yl)-4,5,6,7-tétrahydro-2H-pyrazolo[3,4-c]pyridine | 3.9 mg | 2.62 | 322.27 |

### Exempte 10 : Préparation d'une librairie d'urées

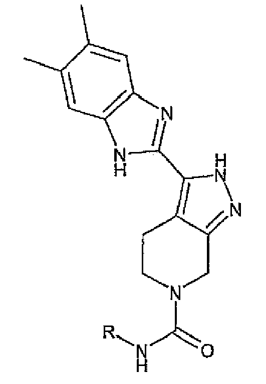

La librairie d'urées peut être préparée de la façon suivante :
120 mg de chlorhydrate de la 3-(5,6-diméthyl-1H-benzimidazol-2-yl)-4,5,6,7-tétrahydro-2H-pyrazolo[3,4-c]pyridine sont mis en suspension dans 3.6 mL de tétrahydrofurane en présence de 190 µL de triéthylamine et la suspension obtenue est distribuée dans chacun des 9 tubes à essais, à raison de 300 µL par tube.

Les 9 isocyanates (tableau 10) sont pesés et additionnés dans chacun des 9 tubes à essais.

**Tableau 10 : Isocyanates utilisés**

| Entrée | Nom | Quantité |
|---|---|---|
| 1 | PHENYL ISOCYANATE | 6.7 mg |
| 2 | BENZYL ISOCYANATE | 7.5 mg |
| 3 | 2-CHLOROPHENYL ISOCYANATE | 8.6 mg |
| 4 | 3-CHLOROPHENYL ISOCYANATE | 8.6 mg |
| 5 | 4-CHLOROPHENYL ISOCYANATE | 8.6 mg |
| 6 | N-BUTYL ISOCYANATE | 5.6 mg |
| 7 | 2-THIENYL ISOCYANATE | 7 mg |
| 8 | 2-METHOXYPHENYL ISOCYANATE | 8.4 mg |
| 9 | O-TOLYL ISOCYANATE | 7.5 mg |

Les 9 mélanges réactionnels sont agités par agitation orbitalaire à température ambiante pendant 2 heures puis évaporés à sec.

Les résidus d'évaporation sont placés en solution dans 1 mL de DMSO chacun et pour chaque solution obtenue, un prélèvement de 10 µL est effectué et dilué dans 40 µL de DMSO (Gilson Liquid Handler Quad-Z 215). Chaque échantillon en solution dans le DMSO ainsi obtenu, est analysé par LC/MS (Méthode A).

Les solutions résiduelles sont purifiées par LC/MS (Méthode B). Après purification par LC/MS, les fractions contenant les composés cherchés sont (éventuellement réunies) soit évaporées à sec (entrées 1, 3, 6, 8, 9), soit déposées sur phase SCX (500 mg de phase CUBCX1-HL ; entrées 2, 4, 5, 7). Les phases SCX sont ensuite lavées avec du méthanol puis extraites avec une solution d'ammoniac 2M dans le méthanol. Les solutions d'extraction sont collectées en tubes de verre tarés, évaporées à sec (évaporateur centrifuge Savant AES 2000 ou Genevac HT8), pesées (Mettler Toledo Automated Workstation LA200) et diluées à 10 mM dans le DMSO (Gilson Liquid Handler Quad-Z 215). Chaque solution obtenue est analysée par LC/MS (Méthode A).

Les composés suivants (Tableau 11), ont été isolés et caractérisés par leur temps de rétention et pic moléculaire en Spectrométrie de Masse (Méthode A).

**Tableau 11 : librairie d'urées obtenue**

| Entrée | Nom | Quantité de produit obtenue | Temps de rétention (minutes) | Ion moléculaire détecté |
|---|---|---|---|---|
| 1 | bis-trifluoroacétate du phénylamide de l'acide3-(5,6-diméthyl-1H-benzo-imidazol-2-yl)-2,4,5,7-tétrahydro-pyrazolo[3,4-c]pyridine-6-carboxylique | 14.6 mg | 3.04 | 387.28 |
| 2 | benzylamide de l'acide3-(5,6-diméthyl-1H-benzoimidazol-2-yl)-2,4,5,7-tétrahydro-pyrazolo[3,4-c]pyridine-6-carboxylique | 1.8 mg | 2.78 | 401.29 |
| 3 | bis-trifluoroacétate du (2-chloro-phényl)-amide de l'acide3-(5,6-diméthyl-1H-benzoimidazal-2-yl)-2,4,6,7-tétrahydro-pyrazolo[3,4-c]pyridine-6-carboxylique | 16 mg | 2.92 | 421.25 |
| 4 | (3-chloro-phényl)-amide de l'acide3-(5,6-diméthyl-1H-benzoimidazol-2-yl)-2,4,5,7-tétrahydro-pyrazolo[3,4-c]pyridine-6-carboxylique | 7.9 mg | 3.89 | 421.24 |
| 5 | (4-chloro-phényl)-amide de l'acide3-(5,6-diméthyl-1H-benzoimidazol-2-yl)- 2,4,5,7-tétrahydro-pyrazolo[3,4-c]pyridine-6-carboxylique | 9.8 mg | 3.36 | 421.25 |
| 6 | bis-trifluoroacétate du butylamide de l'acide3-(5,6-diméthyl-1H-benzoimidazol-2-yl)-2,4,5,7-tétrahydro-pyrazolo[3,4-c]pyridine-6-carboxylique | 2.4 mg | 2.8 | 367.31 |
| 7 | thiophen-2-ylamide de l'acide3-(5,6-diméthyl-1H-benzoimidazol-2-yl)-2,4,5,7-tétrahydro-pyrazolo[3,4-c]pyridine-6-carboxylique | 3.8 mg | 2.77 | 393.24 |
| 8 | bis-trifluoroacétate du (2-methoxy-phényl)-amide de l'acide3-(5,6-diméthyl-1H-benzoimidazol-2-yl)-2,4,5,7-tétrahydro-pyrazolo[3,4-c]pyidine-6-carboxylique | 14.4mg | 3.14 | 417.28 |
| 9 | bis-trifluoroacétate du o-tolylamide de l'acide3-(5,6-diméthyl-1H-benzoimidazol-2-yl)-2,4,5,7-tétrahydro-pyrazolo[3,4-c]pyridine-6-carboxylique | 16.2 mg | 2.68 | 401.29 |

### Exemple 11 : 3-(5,6-Diméthy)-1H-benzimidazol-2-yl)-4,5,6,7-tétrahydro-2H-pyrazolo[3,4-c]pyridine

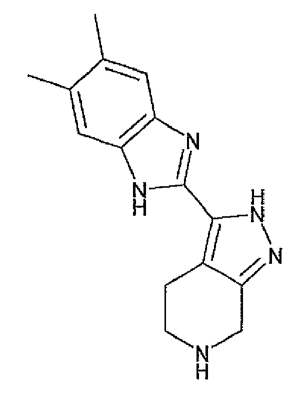

La 3-(5,6-diméthyl-1H-benzimidazol-2-yl)-4,5,6,7-tétrahydro-2H-pyrazolo[3,4-c]pyridine peut être préparée de la façon suivante :
A une solution de 670 mg de 3-(2-amino-4,5-diméthyl-phénylcarbamoyl)-2,4,5,7-tétrahydro-pyrazolo[3,4-c]pyridine-6-carboxylate de tert-butyle dans 9 mL de THF sont additionnés 9 mL d'eau et 2.8 mL d'acide trifluoroacétique. Après 2 heures d'agitation à 80°C, le milieu réactionnel est concentré sous pression réduite. Il est ensuite repris dans de l'eau et le précipité formé est récupéré par filtration sur fritté, lavé avec une solution aqueuse de soude 1N et séché. La phase aqueuse obtenue est alors extraite avec du dichlorométhane, puis la phase organique est séchée sur sulfate de magnésium et concentrée sous pression réduite. Le résidu obtenu et le précipité sont réunis puis mis en solution dans le méthanol avec quelques gouttes de DMF. Cette solution est ensuite déposée sur phase MEGA BE-SCX. La phase SCX est ensuite lavée avec du méthanol puis extraite avec une solution d'ammoniac 2M dans le méthanol. La solution d'extraction obtenue est alors concentrée sous pression réduite.

On obtient ainsi 46 mg de 3-(5,6-diméthyl-1H-bonzimidazol-2-yl)-4,5,6,7-tétrahydro-2H-pyrazolo[3,4-c]pyridine sous forme de poudre beige dont les caractéristiques sont les suivantes :

| | | | |
|---|---|---|---|
| EI : | m/z=267 | m^{+.} | pic de base |
| | m/z=238 | [M - NHCH₂]⁺ | |
| | m/z=209 | [M - C₃H₈N]^{+.} | |

R.M.N. ¹H (300 MHz, (CD₃)₂SO, δ en ppm): 2,31 et 2,32 (2 s: 6H en totalité) ; 2,81 (t large, J = 5 Hz : 2H) ; 2,92 (t large, J = 5 Hz : 2H) ; 3,83 (s large: 2H) ; 7,22 (s large : 1H) ; 7,40 (s large : 1H) ; 12,28 (mf : 1H) ; 12,73 (mf : 1H).

### Exemple 12 : Chlorhydrate de la 3-(5,6-diméthyl-1H-benzimidazol-2-yl)-4,5,6,7-tétrahydro-2H-pyrazolo[3,4-c]pyridine

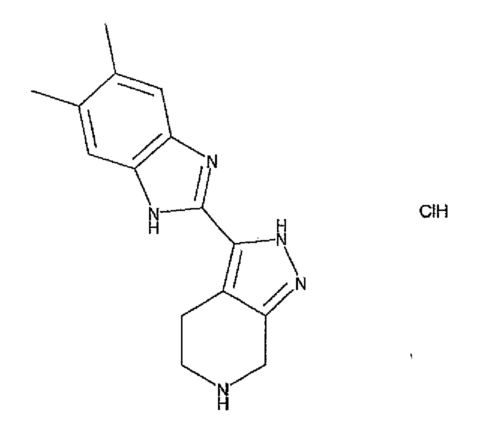

Le chlorhydrate de la 3-(5,6-diméthyl-1H-benzimidazol-2-yl)-4,5,6,7-tétrahydro-2H-pyrazolo[3,4-c]pyridine peut être préparé de la façon suivante :
A une solution de 1.7 g de 3-(2-amino-4,5-diméthyl-phénylcarbamoyl)-2,4,5,7-tétrahydro-pyrazolo[3,4-c]pyridine-6-carboxylate de tert-butyle dans 40 mL d'éthanol est additionné 9 mL d'une solution aqueuse d'acide chlorhydrique 5N. Après 60 heures d'agitation à 80°C, le milieu réactionnel est ramené à température ambiante. Le précipité formé est récupéré par filtration sur fritté et séché. On obtient ainsi 1.04 g de chlorhydrate de la 3-(5,6-diméthyl-1H-benzimidazol-2-yl)-4,5,6,7-tétrahydro-2H-pyrazolo[3,4-c]pyridine sous forme de poudre beige dont les caractéristiques sont les suivantes :

| | | | |
|---|---|---|---|
| EI : | m/z=267 | M^{+.} | pic de base |
| | m/z=238 | [M - CH₂NH]⁺ | |
| | m/z=209 | [M - C₃H₈N]^{+.} | |
| | m/z=36 | [HCl]⁺ | |

R.M.N. ¹H (300 MHz, (CD₃)₂SO avec ajout de quelques gouttes de CD₃COOD, δ en ppm) : 2,40 (s : 6H) ; 3,23 (t large, J = 5,5 Hz : 2H) ; 3,45 (t, J = 5,5 Hz : 2H) ; 4,45 (s : 2H) ; 7,54 (s : 2H).

Le 3-(2-amino-4,5-diméthyl-phénylcarbamoyl)-2,4,5,7-tétrahydro-pyrazolo [3,4-c]pyridine-6-carboxylate de tert-butyle peut être préparé de la façon suivante :
A une solution de 3 g de l'acide 2,4,5,7-tétrahydro-pyrazolo[3,4-c]pyridine-6-carboxylate de tert-butyle-3-carboxylique dans 50 mL de DMF anhydre, sont additionnés, à température ambiante, 8.5 g de HBTU ainsi que 2.9 g de diisopropyléthylamine. Après vingt minutes d'agitation à température ambiante, sont additionnés 3.06 g de 4,5-diamino-o-xylène. Après 60 heures d'agitation à température ambiante, le milieu réactionnel est dilué dans 3 L d'une solution aqueuse de NaHCO3 à pH supérieur à 7, contenant 20 g de NaCl. La phase aqueuse est extraite trois fois avec 1 L d'acétate d'éthyle, puis les phases organiques rassemblées sont séchées sur sulfate de magnésium et concentrées sous pression réduite. Le résidu brut obtenu est repris dans 150 mL de dichlorométhane et l'insoluble est éliminé par filtration sur fritté. Le filtrat est alors concentré sous pression réduite et purifié par chromatographie sur silice (20-45 µm Amicon) avec un gradient de 50 à 100 % d'acétate d'éthyle dans le cyclohexane. Les fractions contenant le produit recherché sont réunies et concentrées sous pression réduite. On obtient ainsi 4.37 g de 3-(2-amino-phénylcarbamoyl)-2,4,5,7-tétrahydro-pyrazolo[3,4-c]pyridine-6-carboxylate de tert-butyle sous forme d'une poudre beige dont les caractéristiques sont les suivantes :

| | | | |
|---|---|---|---|
| El: | m/z=385 | M^{+.} | pic de base |
| | m/z=329 | [M - C₄H₈]^{+.} | |
| | m/z=312 | [M - C₄H₉O]⁺ | |
| | m/z=57 | [ C₄H₉]⁺ | |

R.M.N. ¹H (300 MHz, (CD₃)₂SO, δ en ppm) : 1,46 (s : 9H) ; 2,10 et 2,12 (2 s : 3H chacun) ; 2,77 (mt : 2H) ; 3,58 (t, J = 5,5 Hz : 2H) ; 4,53 (s : 2H) ; 4,57 (mf : 2H) ; 6,60 (s : 1H) ; 7,14 (s large: 1H) ; 9,10 (mf : 1H) ; 13,08 (mf : 1H).

### Exemple 13 : Chlorhydrate de la 3-(1H-benzimidazol-2-yl)-4,5,6,7-tétrahydro-2H-pyrazolo[3,4-c]pyridine

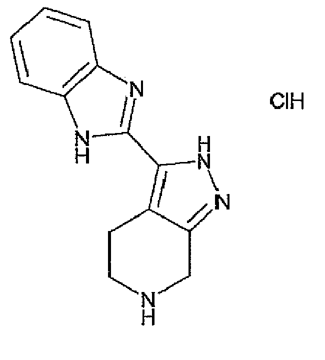

Le chlorhydrate de la 3-(1H-benzimidazol-2-yl)-4,5,6,7-tétrahydro-2H-pyrazolo[3,4-c]pyridine peut être préparé de la façon suivante :
A une solution de 200 mg de 3-(2-amino-phénylcarbamoyl)-2,4,5,7-tétrahydro-pyrazolo[3,4-c]pyridine-6-carboxylate de tert-butyle dans 1 mL d'éthanol est additionné 2.2 mL d'une solution aqueuse d'acide chlorhydrique 5 N. Après 20 heures d'agitation à 80°C, le milieu réactionnel est ramené à température ambiante. L'insoluble est éliminé par filtration sur fritté et le filtrat est concentré sous pression réduite. On obtient ainsi 84 mg de chlorhydrate de la 3-(1H-benzimidazol-2-yl)-4,5,6,7-tétrahydro-2H-pyrazolo[3,4-c]pyridine sous forme de poudre orangée dont les caractéristiques sont les suivantes :
LC/MS (Méthode A) : ion moléculaire détecté : 240.26 ; temps de rétention = 1.68 minutes

Le 3-(2-amino-phénylcarbamoyl)-2,4,5,7-tétrahydro-pyrazolo[3,4-c]pyridine-6-carboxylate de tert-butyle peut être préparé de la façon suivante :
A une solution de 150 mg de l'acide 2,4,5,7-tétrahydro-pyrazolo[3,4-c]pyridine-6-carboxylate de tert-butyle-3-carboxylique dans 1 mL de DMF anhydre, sont additionnés, à température ambiante, 425 mg de HBTU ainsi que 145 mg de diisopropyléthylamine. Après vingt minutes d'agitation à température ambiante, sont additionnés 121 mg d'orthophénylènediamine. Après 20 heures d'agitation à température ambiante, le milieu réactionnel est dilué dans 100 mL d'eau et 50 mL d'acétate d'éthyle.. La phase aqueuse est extraite trois fois avec 50 mL d'acétate d'éthyle, puis les phases organiques rassemblées sont séchées sur sulfate de magnésium et concentrées sous pression réduite. Le résidu brut obtenu est purifié par HPLC (phase inverse C18 Lichroprep 12 µm) avec un gradient linéaire de 5 à 95 % d'acétonitrile contenant 0,07 % (v/v) d'acide trifluoroacétique dans l'eau contenant 0,07 % (v/v) d'acide trifluoroacétique, à un débit de 10 mL/mn. Les fractions contenant le produit recherché sont réunies et déposées sur phase MEGA BE-SCX. La phase SCX est ensuite lavée avec du méthanol puis extraite avec une solution d'ammoniac 2M dans le méthanol. La solution d'extraction obtenue est alors concentrée sous pression réduite. On obtient ainsi 200 mg de 3-(2-amino-phénylcarbamoyl)-2,4,5,7-tétrahydro-pyrazolo[3,4-c]pyridine-6-carboxylate de tert-butyle sous forme d'une poudre beige dont les caractéristiques sont les suivantes :
LC/MS (Méthode A) : ion moléculaire détecté : 355,34 ; temps de rétention = 3,19 minutes.

### Exemple 14 : Préparation d'une librairie de sulfonamides

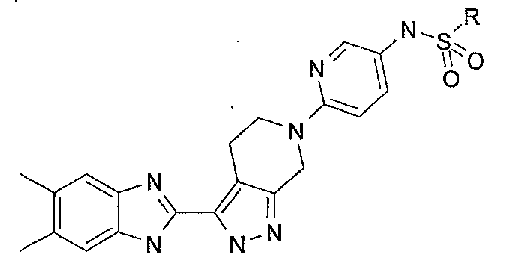

La librairie de sulfonamides peut être préparée de la façon suivante :
40 mg de 6-[3-(5,6-diméthyl-1H-benzimidazol-2-yl)-2,4,5,7-tétrahydro-pyrazolo[3,4-c]pyridin-3-yl]-pyridin-3-ylamine sont mis en suspension dans 2 mL de dichlorométhane et la solution obtenue est distribuée dans 4 tubes à essais, à raison de 500 µL par tube.

Les 4 chlorures de sulfonyle (tableau 12) sont pesés et additionnés dans chacun des 4 tubes à essais, suivis de 15.6 µL de triéthytamine.

**Tableau 12 : Chlorures de sulfonyle utilisés**

| Entrée | Nom | Quantité |
|---|---|---|
| 1 | CHLORURE DE THIOPHENE-2-SULFONYLE | 5.6 mg |
| 2 | CHLORURE DE 4-METHOXYBENZENESULFONYLE | 6.3 mg |
| 3 | CHLORURE DE 2-CHLOROBENZENESULFONYLE | 6.4 mg |
| 4 | CHLORURE DE 2-METHOXY-4-METHYLBENZENESULPHONYLE | 6.8 mg |

Les 4 mélanges réactionnels sont agités par agitation orbitalaire à 40°C pendant 15 heures.
Pour chaque mélange réactionnel, un prélèvement de 5 µL est effectué et dilué dans 100 µL de DMSO (Gilson Liquid Handler Quad-Z 215). Chaque échantillon en solution dans le DMSO ainsi obtenu, est analysé par LC/MS (Méthode A).

Les 4 mélanges réactionnels sont ensuite évaporés à sec et les résidus d'évaporation sont placés en solution dans 500 µL de DMSO chacun et les solutions obtenues sont purifiées par LC/MS (Méthode B). Après purification par LC/MS, les fractions contenant les composés cherchés sont (éventuellement réunies) déposées sur phase SCX (500 mg de phase CUBCX1-HL). Les phases SCX sont ensuite lavées avec du méthanol puis extraites avec une solution d'ammoniac 2M dans le méthanol. Les solutions d'extraction sont collectées en tubes de verre tarés, évaporées à sec (évaporateur centrifuge Savant AES 2000 ou Genevac HT8), pesées (Mettler Toledo Automated Workstation LA200) et diluées à 10 mM dans le DMSO (Gilson Liquid Handler Quad-Z 215). Chaque solution obtenue est analysée par LC/MS (Méthode A).

Les composés suivants (Tableau 13), ont été isolés et caractérisés par leur temps de rétention et pic moléculaire en Spectrométrie de Masse (Méthode A).

**Tableau 13 : librairie de sulfonamides obtenue**

| Entrée | Nom | Quantité de produit obtenue | Temps de rétention (minutes) | Ion moléculaire détecté |
|---|---|---|---|---|
| 1 | N-{6-[3-(5,6-diméthy)-1H-benzimldazol-2-yl)-2,4,5,7-tétrahydro-pyrazolo[3,4-c]pyridin-6-yl]-pyridin-3-yl}-thiophène-2-sulfonamide | 2.9 mg | 3.07 | 506.21 |
| 2 | N-{6-[3-(5,6-Diméthyl-1H-benzimidazol-2-yl)-2,4,5,7-tétrahydro-pyrazolo[3,4-c]pyridin-6-yl]-pyridin-3-yl}-4-méthoxy-benzènesulfonamide | 3.0 mg | 3.20 | 530.25 |
| 3 | 2-Chloro-N-{6-[3-(5,6-diméthyl-1H-benzimidazol-2-yl)-2,4,5,7-tétrahydro-pyrazolo[3,4-c]pyridin-6-yl]-pyridin-3-yl}-benzènesulfonamide | 3.0 mg | 3.38 | 534.21 |
| 4 | N-{6-[3-(5,6-Diméthyl-1H-benzimidazol-2-yl)-2,4,5,7-tétrahydro-pyrazolo[3,4-c]pyridin-6-yl]-pyridin-3-yl}-2-méthoxy-4-methyl-benzènesulfonamide | 3.8 mg | 3.38 | 544.26 |

### Exemple 15 : 6-[3-(5,6-Diméthyl-1H-benzimidazol-2-yl)-2,4,5,7-tétrahydro-pyrazolo[3,4-c]pyridin-6-yl]-pyridin-3-ylamine

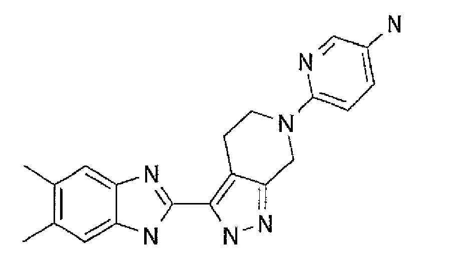

La 6-[3-(5,6-diméthyl-1H-benzimidazol-2-yl)-2,4,5,7-tétrahydro-pyrazolo[3,4-c]pyridin-6-yl]-pyridin-3-ylamine peut être préparée de la façon suivante :
A une solution de 545 mg de 3-(5,6-diméthyl-1H-benzimidazol-2-yl)-6-(5-nitro-pyridin-2-yl)-4,5,6,7-tétrahydro-2H-pyrazolo[3,4-c]pyridine dans 60 mL d'éthanol est additionné 55 mg de Pd/CaCO3 10%. Après 15 heures d'agitation à 35°C sous 3 bars d'hydrogène, le milieu réactionnel est ramené à température ambiante, filtré sur célite puis concentré sous pression réduite. On obtient ainsi 300 mg de 6-[3-(5,6-diméthyl-1H-benzimidazol-2-yl)-2,4,5,7-tétrahydro-pyrazolo[3,4-c]pyridin-6-yl]-pyridin-3-ylamine sous forme de poudre marron dont les caractéristiques sont les suivantes :

| | | | |
|---|---|---|---|
| El | m/z=359 | M^{+.} | pic de base |
| | m/z=266 | (M - C₅H₅N₂)⁺ | |

### Exemple 16 : 3-(5,6-Diméthyl-1H-benzimidazol-2-yl)-6-(5-nitro-pyridin-2-yl)-4,5,6,7-tétrahydro-2H-pyrazolo[3,4-c]pyridine

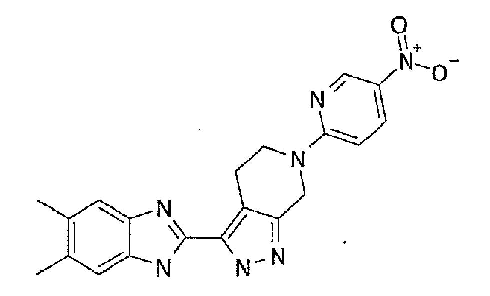

La 3-(5,6-diméthyl-1H-benzimidazol-2-yl)-6-(5-nitro-pyridin-2-yl)-4,5,6,7-tétrahydro-2H-pyrazolo[3,4-c]pyridine peut être préparée de la façon suivante :
A une solution de 500 mg de chlorhydrate de la 3-(5,6-diméthyl-1H-benzimidazol-2-yl)-4,5,6,7-tétrahydro-2H-pyrazolo[3,4-c]pyridine dans 5 mL de diméthylformamide sont additionnés 287 mg de 2-chloro-5-nitropyridine et 500 mg de carbonate de potassium. Après 20 heures d'agitation à température ambiante, le milieu réactionnel est additionné à 50 ml d'eau. Le précipité formé est récupéré par filtration sur fritté, lavé avec 3 fois 15 mL d'eau puis séché sous pression réduite. On obtient ainsi 548 mg de 3-(5,6-diméthyl-1H-benzimidazol-2-yl)-6-(5-nitro-pyridin-2-yl)-4,5,6,7-tétrahydro-2H-pyrazolo[3,4-c]pyridine sous forme de poudre jaune dont les caractéristiques sont les suivantes :

| | | | |
|---|---|---|---|
| El : | m/z=389 | M^{+.} | pic de base |
| | m/z=266 | (M - C₅H₃N₂O₂)⁺ | |

### Exemple 17: 6-{5-[3-(2-Fluoro-5-trifluorométhyl-phényl)-uréidol-pyridin-2-yl}-4,5,6,7-tétrahydro-2H-pyrazolo[3,4-c]pyidine-3-carboxamide

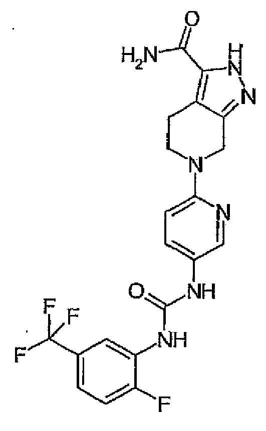

Le 6-{5-[3-(2-Fluoro-5-trifluorométhyl-phényl)-uréido]-pyridin-2-yl}-4,5,6,7-tétrahydro-2H-pyrazolo[3,4-c]pyridine-3-carboxamide peut être préparé de la façon suivante à partir du 6-(5-tert-butoxycarbonylamino-pyridin-2-yl)-4,5,6,7-tétrahydro-2H-pyrazolo[3,4-c]pyridine-3-carboxylate d'éthyle:

L'ester éthylique du 6-(5-tert-butoxycarbonylamino-pyridin-2-yl)-4,5,6,7-tétrahydro-2H-pyrazolo[3,4-c]pyridine-3-carboxylate d'éthyle est transformé en carboxamide par acidification par une solution d'ammoniaque et conduit à l'obtention de 6-(5-tert-butoxycarbonylamino-pyridin-2-yl)-4,5,6,7-tétrahydro-2H-pyrazolo[3,4-c]pyridine-3-carboxamide.
El : m/z=358

Le groupement amine du 6-(5-tert-butoxycarbonylamino-pyridin-2-yl)-4,5,6,7-tétrahydro-2H-pyrazolo[3,4-c]pyridine-3-carboxamide est déprotégé en milieu acide (acide trifluoroacétique dans le dichlorométhane) et conduit à l'obtention de 6-(5-amino-pyridin-2-yl)-4,5,6,7-tétrahydro-2H-pyrazolo[3,4-c]pyridine-3-carboxamide.
El : m/z= 258

La fonction urée est introduite sur le 6-(5-amino-pyridin-2-yl)-4,5,6,7-tétrahydro-2H-pyrazolo[3,4-c]pyridine-3-carboxamide selon la méthode décrite à l'exemple 1 en utilisant le 2-fluoro-5-(trifluorométhyl)phénylisocyanate, et conduit à l'obtention du 6-{5-[3-(2-fluoro-5-trifluorométhylphényl)-uréido]-pyridin-2-yl}-4,5,6,7-tétrahydro-2H-pyrazolo[3,4-c]pyridine-3-carboxamide.
EI: m/z=463

### Exemple 18 : 6-(5-tert-Butoxycarbonylamino-pyridin-2-yl)-4,5,6,7-tétrahydro-2H-pyrazolo[3,4-c]pyridine-3-carboxylate d'éthyle

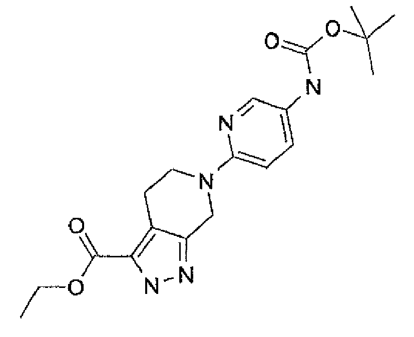

Le 6-(5-tert-Butoxycarbonylamino-pyridin-2-yl)-4,5,6,7-tétrahydro-2H-pyrazolo [3,4-c]pyridine-3-carboxylate d'éthyle peut être préparé de la façon suivante :
A une solution de 50 mg de 6-(5-nitro-pyridin-2-yl)-4,5,6,7-tétrahydro-2H-pyrazolo[3,4-c]pyridine-3-carboxylate d'éthyle dans 6 mL de méthanol sont additionnés 5 mg de Pd/C 10 % et 38 mg de di-tert-butyldicarbonate. Après 12 heures d'agitation à température ambiante sous 3 bars d'hydrogène, le milieu réactionnel est filtré sur célite puis concentré sous pression réduite. Le brut réactionnel obtenu est purifié par chromatographie flash (SiO₂, CH₂Cl₂/MeOH gradient 75/25 à 25/75). On obtient ainsi 20 mg de 6-(5-tert-Butoxycarbonylamino-pyridin-2-yl)-4,5,6,7-tétrahydro-2H-pyrazolo[3,4-c]pyridine-3-carboxylate d'éthyle sous forme de poudre blanche dont les caractéristiques sont les suivantes :

| | | | |
|---|---|---|---|
| El: | m/z=387 | m^{+.} | |
| | m/z=331 | (M - C₄H₈)^{+.} | pic de base |
| | m/z=286 | (m/z=331 - CO₂H)⁺ | |
| | m/z=194 | C₉H₁₂N₃O₂⁺ | |
| | m/z=57 | C₄H₉⁺ | |

### Exemple 19 : 6-(5-Nitro-pyridin-2-yl)-4,5,6,7-tétrahydro-2H-pyrazolo[3,4-c]pyridine-3-carboxlate d'éthyle

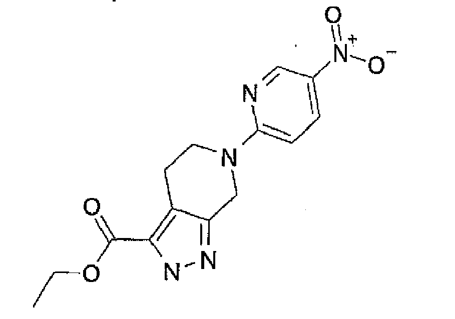

Le 6-(5-Nitro-pyridin-2-yl)-4,5,6,7-tétrahydro-2H-pyrazolo[3,4-c]pyridine-3-carboxylate d'éthyle peut être préparé de la façon suivante :
A une solution de 1 g de trifluoroacétate de la 3-(5,6-diméthyl-1H-benzimidazol-2-yl)-4,5,6,7-tétrahydro-2H-pyrazolo[3,4-c]pyridine dans 10 mL de pyridine sont additionnés 522 mg de 2-chloro-5-nitropyridine. Après 20 heures d'agitation à température ambiante, le milieu réactionnel est concentré sous pression réduite. Le précipité formé est récupéré par filtration sur fritté, lavé avec 3 fois 15 mL d'eau, puis séché sous pression réduite. Le brut réactionnel obtenu est purifié par chromatographie flash (SiO₂, cyclohexane/AcOEt gradient 75/25 à 25/75). On obtient ainsi 450 mg de 6-(5-Nitro-pyridin-2-yl)-4,5,6,7-tétrahydro-2H-pyrazolo[3,4-c]pyridine-3-carboxylate d'éthyle sous forme de poudre jaune dont les caractéristiques sont les suivantes :

| | | | |
|---|---|---|---|
| El : | m/z=317 | M^{+.} | pic de base |
| | m/z=271 | (M - NO₂)^{+.} | |
| | m/z=194 | (M - C₅H₃N₂O₂)⁺ | |
| | m/z=148 | (m/z=194 - C₂H₆O)⁺ | |

### Exempte 20 : Trifluoroacétate de 4,5,6,7-tétrahydro-2H-pyrazolo[3,4-c]pyridine-3-carboxylate d'éthyle

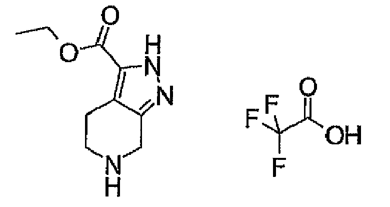

Le trifluoroacétate du 4,5,6,7-tétrahydro-2H-pyrazolo[3,4-c]pyridine-3-carboxylate d'éthyle peut être préparé de la façon suivante :
A une solution de 3 g de 2,4,5,7-tétrahydro-pyrazolo[3,4-c]pyridyl-3,6-dicarboxylate de 6-tert-butyle 3-éthyle dans 50 mL de tétrahydrofurane, sont additionnés 50 mL d'eau suivis de 12 mL d'acide trifluoroacétique. Après 2 heures d'agitation au reflux, le milieu réactionnel est ramené à température ambiante et est additionné d'une solution aqueuse saturée de Na₂CO₃ jusqu'à l'obtention d'un pH basique. La phase aqueuse obtenue est extraite 3 fois avec de l'acétate d'éthyle. Les phases organiques rassemblées sont séchées sur sulfate de magnésium et concentrées sous pression réduite. On obtient ainsi 1.49 g de trifluoroacétate du 4,5,6,7-tétrahydro-2H-pyrazolo[3,4-c]pyridine-3-carboxylate d'éthyle dont les caractéristiques sont les suivantes :

| | | | |
|---|---|---|---|
| EI: | m/z=195 | M^{+.} | |
| | m/z=166 | (M - CH₃N)^{+.} | pic de base |
| | m/z=138 | (M - C₃H₇N)^{+.} | |
| | m/z=120 | (m/z=166 - C₂H₆O)^{+.} | |
| | m/z=92 | (m/z=120 - CO)^{+.} | |

### Mesures du potentiel d'inhibition des produits à l'encontre de l'activité des kinases Tie2 et KDR :

L'activité inhibitrice des produits à l'encontre des kinases Tie2 et KDR est testée selon les protocoles expérimentaux décrits ci-dessous.

### 1. Tie2

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.
L'effet inhibiteur des molécules est déterminé dans un test de phosphorylation de PLC par Tie2 en présence de GST-Tie2 purifiée à environ 80 % d'homogénéité. Le substrat est composé des fragments SH2-SH3 de la PLC exprimée sous forme de protéine de fusion GST.
L'activité kinase de Tie2 est mesurée dans un tampon MOPS 20mM pH 7.2, contenant 10 mM MgCl₂, 10 mM MnCl₂, 1 mM DTT, 10 mM de glycérophosphate. Dans une plaque 96 puits FlashPlate maintenue sur glace, on dépose un mélange réactionnel composé de 70 µl de tampon kinase contenant 100 ng d'enzyme GST-Tie2 par puits. Ensuite 10 µl de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi d'³³P[ATP]. Après 1 heure d'incubation à 37°C, la réaction est stoppée en ajoutant 1 volume (100 µl) d'EDTA à 200 mM. Après élimination du tampon d'incubation, les puits sont lavés trois fois avec 300 µl de PBS. La radioactivité est mesurée sur un MicroBeta1450 Wallac.
L'inhibition de l'activité Tie2 est calculée et exprimée en pourcentage d'inhibition par rapport à l'activité contrôle déterminée en l'absence de composé.

### 2. KDR

L'effet inhibiteur des composés est déterminé dans un test de phosphorylation de substrat par l'enzyme KDR *in vitro* par une technique de scintillation (plaque 96 puits, NEN).
Le domaine cytoplasmique de l'enzyme KDR humaine a été cloné sous forme de fusion GST dans le vecteur d'expression baculovirus pFastBac. La protéine a été exprimée dans les cellules SF21 et purifiée à environ 60 % d'homogénéité.

L'activité kinase de KDR est mesurée dans 20 mM MOPS, 10 mM MgCl2, 10 mM MnCl2, 1 mM DTT, 2.5 mM EGTA, 10 mM b-glycérophosphate, pH = 7.2, en présence de 10 mM MgCl₂, 100 µM Na₃VO₄, 1 mM NaF. 10 µl du composé sont ajoutés à 70 µl de tampon kinase contenant 100 ng d'enzyme KDR à 4°C. La réaction est lancée en ajoutant 20 µl de solution contenant 2 µg de substrat (fragment SH2-SH3 de la PLC□ exprimée sous forme de protéine de fusion GST), 2 µCi γ ³³P[ATP] et 2 µM ATP froid. Après 1 heure d'incubation à 37°C, la réaction est stoppée en ajoutant 1 volume (100 µl) de 200 mM EDTA. Le tampon d'incubation est retiré, et les puits sont lavés trois fois avec 300 µl de PBS. La radioactivité est mesurée dans chaque puits en utilisant un compteur de radioactivité Top Count NXT (Packard).
Le bruit de fond est déterminé par la mesure de la radioactivité dans quatre puits différents contenant l'ATP radioactif et le substrat seul.
Un contrôle d'activité totale est mesuré dans quatre puits différents contenant tous les réactifs (γ³³P-[ATP], KDR et substrat PLCγ) mais en l'absence de composé.
L'inhibition de l'activité KDR avec le composé de l'invention est exprimée en pourcentage d'inhibition de l'activité contrôle déterminée en l'absence de composé.
Le composé SU5614 (Calbiochem) (1 µM) est inclus dans chaque plaque comme contrôle d'inhibition.

### Résultants :

| | Tie2 | | KDR | |
|---|---|---|---|---|
| | % Inhib at 10µM (FRX) | | % Inhib at 10µM | |
| Chemistry | Assay 1 | Assay 2 | Assay 1 | Assay 2 |
| 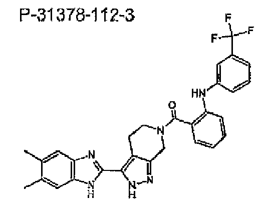 | 78,7 | 79,1 | 59,0 | 56,5 |
| 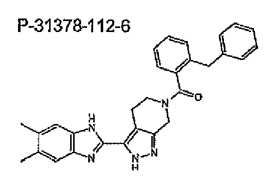 | 92,7 | 93,8 | 71,4 | 68,0 |
| 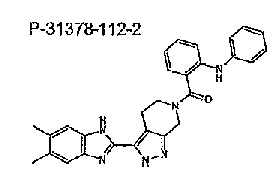 | 92,6 | 92,6 | 98,1 | 97,4 |
| 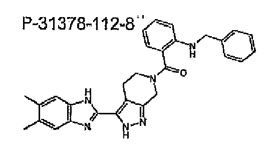 | 87,8 | 93,3 | 86,8 | 89,0 |
| 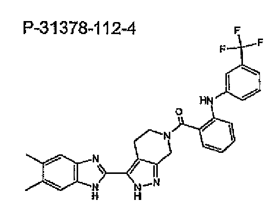 | 83,1 | 87,2 | 56,2 | 50,1 |
| 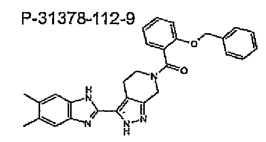 | 82,6 | 85,9 | 53,2 | 45,1 |
| 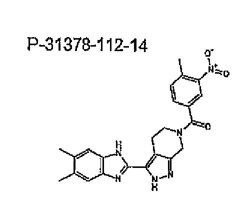 | 69,7 | 73,8 | 19,5 | 13,0 |
| 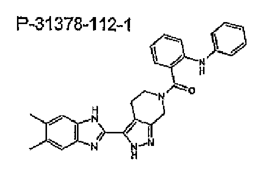 | 83,1 | 88,1 | 96,5 | 96,7 |
| 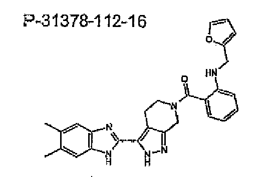 | 88,7 | 88,3 | 90,2 | 89,1 |
| 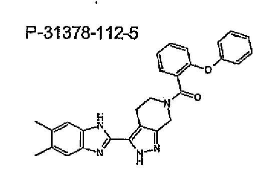 | 87,1 | 86,4 | 94,7 | 95,4 |
| 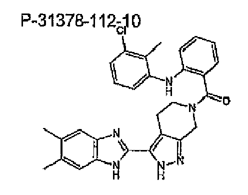 | 90,6 | 87,9 | 42,3 | 28,7 |
| 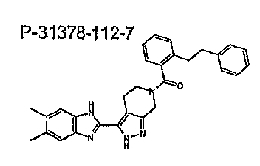 | 92,7 | 92,0 | 68,6 | 64,3 |
| 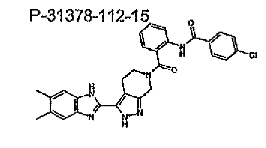 | 80,1 | 83,8 | 75,5 | 77,4 |

| Chemistry | KDR % Inhib. 10µM | Tie2 % Inhib. 10µM |
|---|---|---|
| 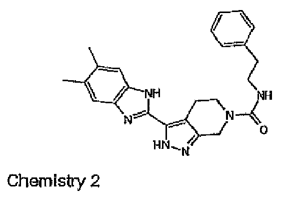 | 77,20 | |
| 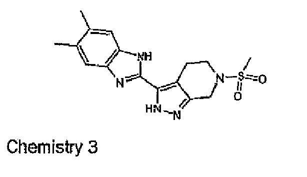 | 78,10 | |
| 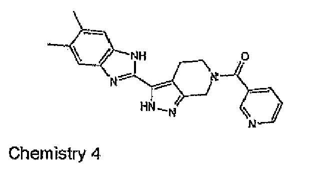 | 25,40 | |
| 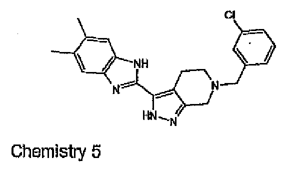 | 88,80 | |
| 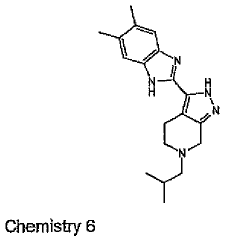 | 2,10 | 41,2 |
| 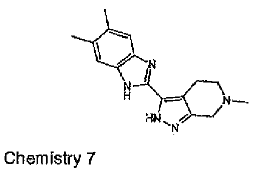 | 12,15 | 48,6 |
| 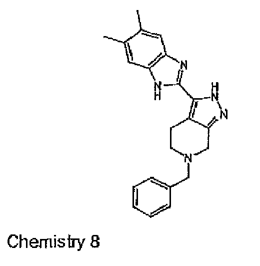 | 85,45 | 64,3 |
| 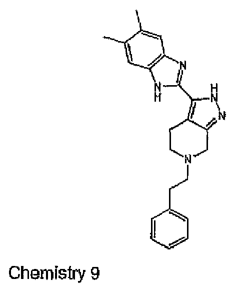 | 15,45 | 56,7 |
| 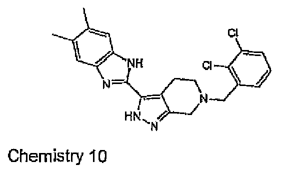 | 79,55 | 83,9 |
| 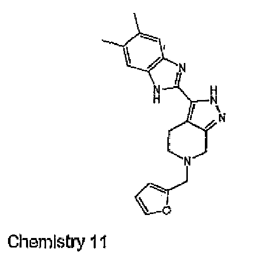 | 70,35 | 66,1 |
| 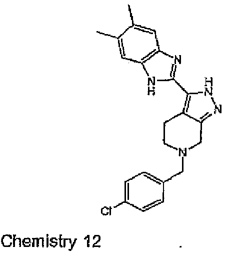 | 69,05 | 60,3 |
| 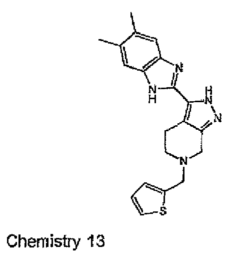 | 84,50 | 41,5 |
| 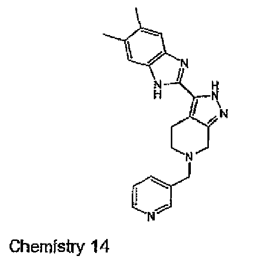 | 14,85 | 50,5 |
| 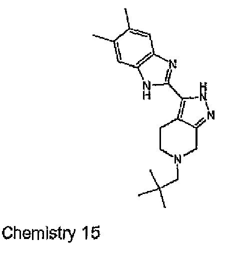 | 39,30 | 69,4 |
| 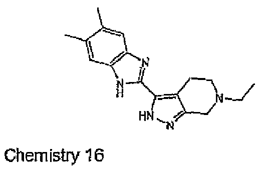 | -5,75 | 36,4 |
| 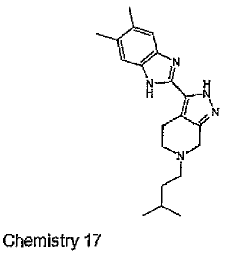 | -2,90 | 50,7 |
| 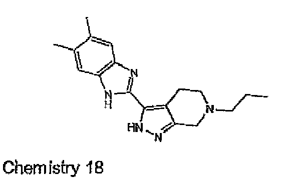 | -3,70 | 55,0 |
| 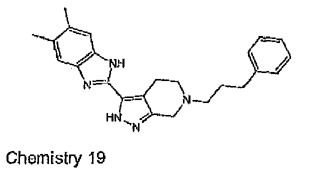 | 17,90 | 68,1 |
| 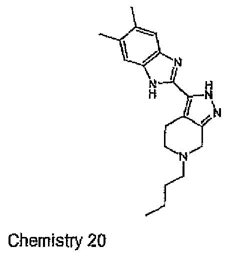 | 1,65 | 47,6 |
| 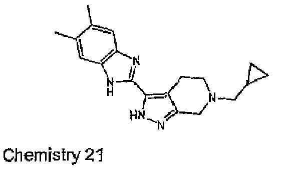 | 1,85 | 33,0 |
| 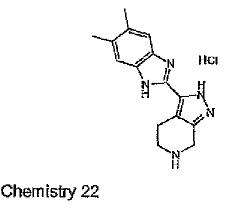 | 3,65 | 24,9 |
| 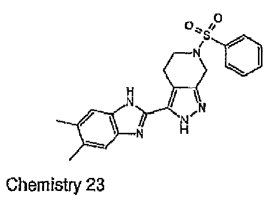 | 48 | 92,5 |
| 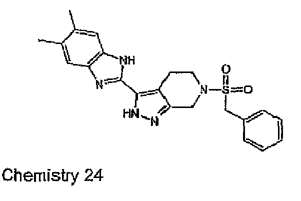 | 70,75 | 91,5 |
| 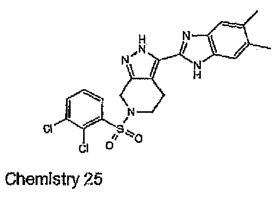 | 31,85 | 89,1 |
| 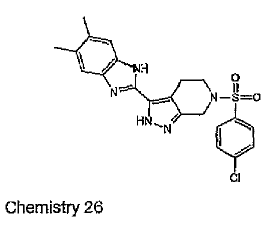 | 27,75 | 89,1 |
| 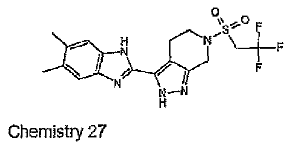 | 22,95 | 65,4 |
| 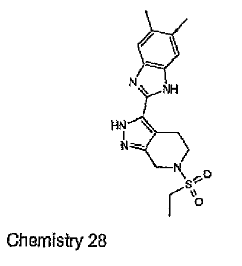 | 16,50 | 78,5 |
| 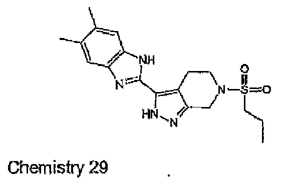 | 13,35 | 47,5 |
| 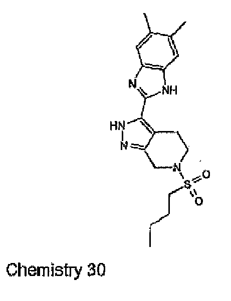 | 15,30 | 44,2 |
| 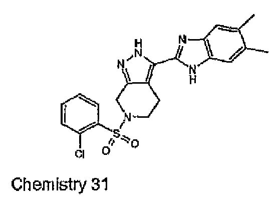 | 45,70 | 93,2 |
| 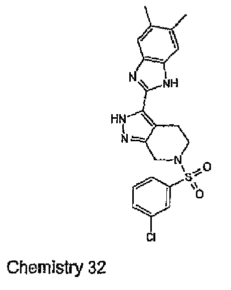 | 46,45 | 91,5 |
| 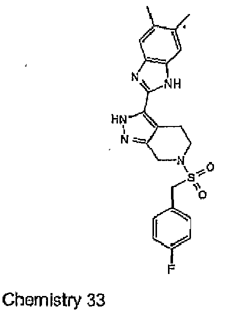 | 57,70 | 95,9 |
| 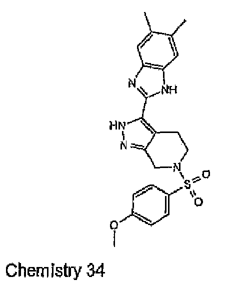 | 18,15 | 84,7 |
| 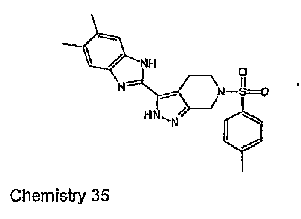 | 27,40 | 88,8 |
| 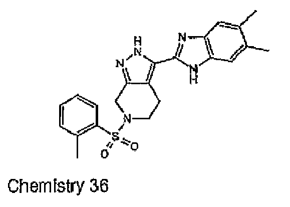 | 49,40 | 90,8 |
| 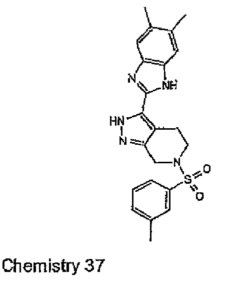 | 41,20 | 88,3 |
| 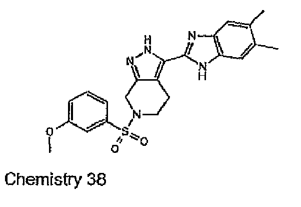 | 20,85 | 84,6 |
| 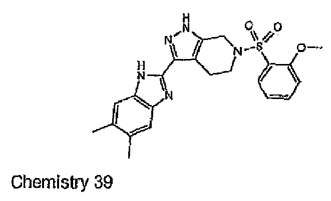 | 30,90 | 97,9 |
| 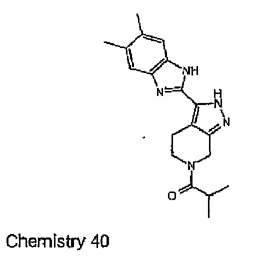 | 14,40 | 83,4 |
| 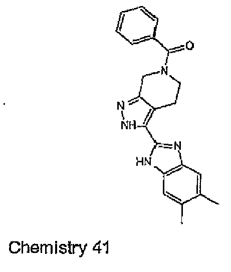 | 72,50 | 86,3 |
| 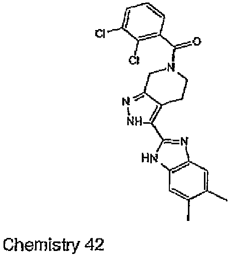 | 52,65 | 97,4 |
| 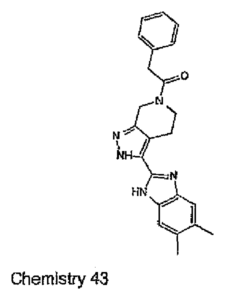 | 10,90 | 84,4 |
| 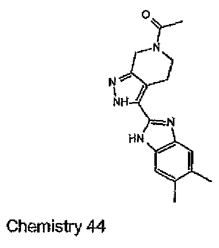 | 41,10 | 90,8 |
| 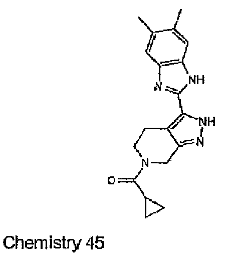 | 33,40 | 94,1 |
| 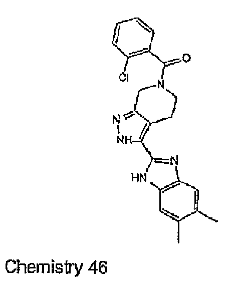 | 73,05 | 96,7 |
| 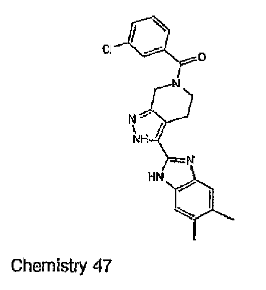 | 78,55 | 87,7 |
| 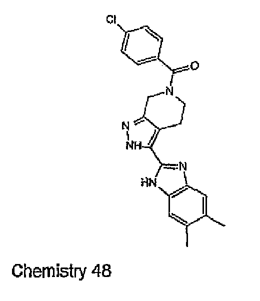 | 41,95 | 83,8 |
| 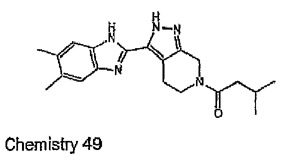 | 12,20 | 84,8 |
| 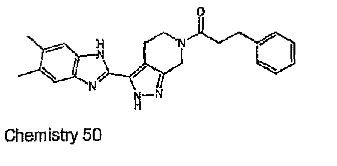 | 74,75 | 82,4 |
| 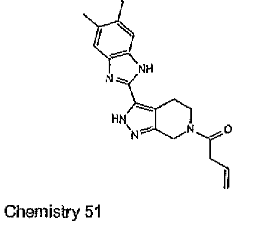 | 38,70 | 72,9 |
| 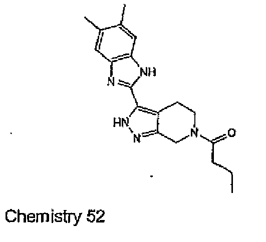 | 29,35 | 89,3 |
| 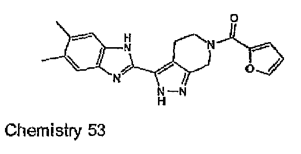 | 74,55 | 92,7 |
| 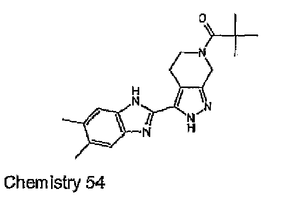 | 11,25 | 81,3 |
| 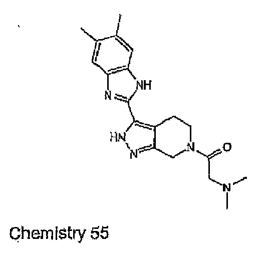 | 15,95 | 65,4 |
| 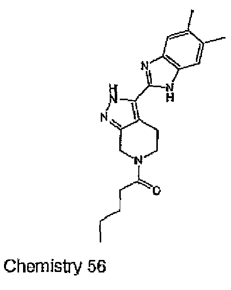 | 46,65 | 92,5 |
| 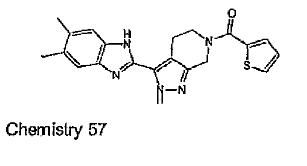 | 75,15 | 94,6 |
| 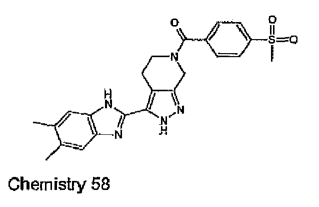 | 27,90 | 82,3 |
| 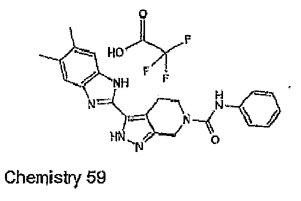 | 17,6 | 33,2 |
| 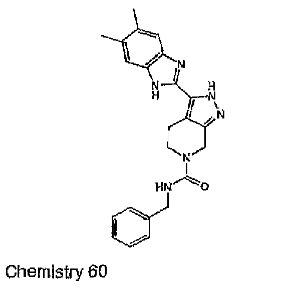 | 33,55 | 64,85 |
| 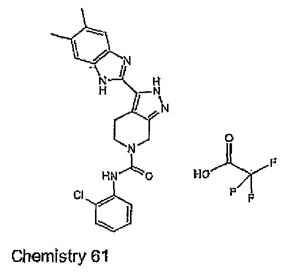 | 12,7 | 3,75 |
| 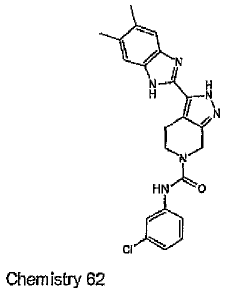 | 12,85 | 21 |
| 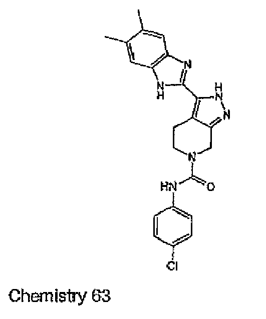 | -0,3 | 23,75 |
| 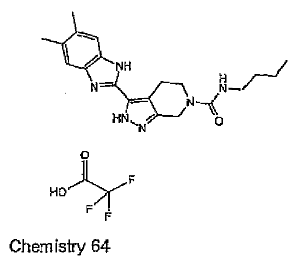 | 18 | 38,5 |
| 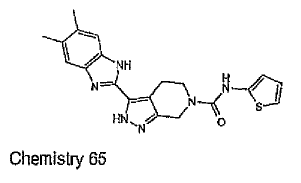 | 63,3 | 85,9 |
| 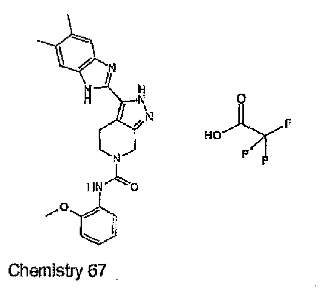 | 8,1 | 18,05 |
| 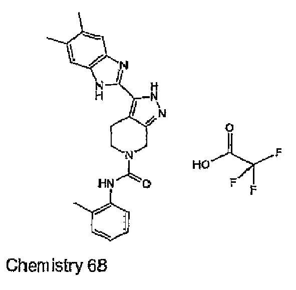 | 19,5 | 19,1 |
| 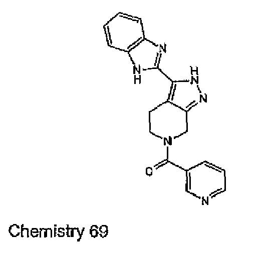 | 18,55 | 59,3 |
| 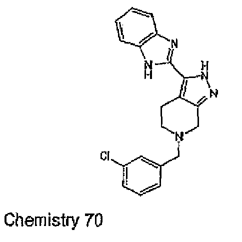 | 92,1 | 65,5 |
| 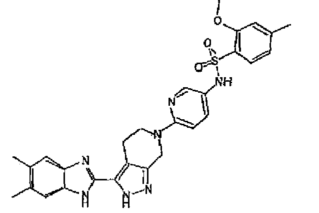 | | 98 |
| 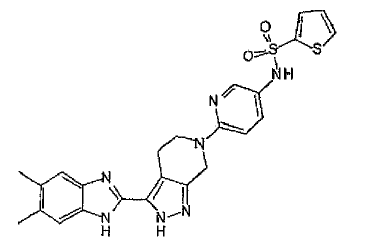 | | 100 |
| 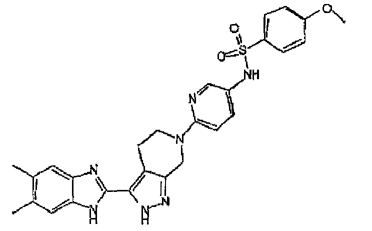 | | 99 |
| 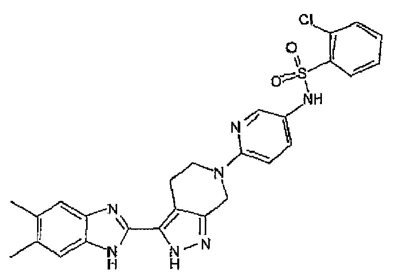 | | 100 |

## Revendications

1. Produit de formule générale (III) suivante : 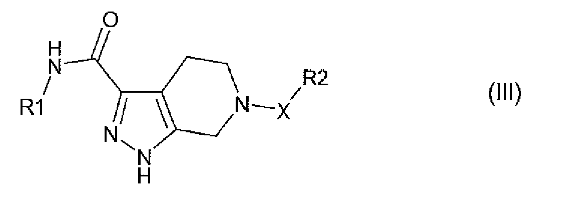 et ses tautomères, **caractérisé en ce que** :
X est choisi parmi liaison, CH₂, CO, SO₂, CONH, COO ;
R1 est choisi parmi alkyle, cycloalkyle, hétérocyclyle, aryle, hétéroaryle, éventuellement substitué ;
R2 est H ou choisi parmi alkyle, alkylène, cycloalkyle, hétérocyclyle, aryle, hétéroaryle, éventuellement substitué ;
et **en ce que** les substituants sont indépendamment choisis parmi R3, O-R3, halogène, NO₂ SO₂-R3, CO-R3, SO₂NH-R3, CONH-R3, N-(R3)₂, NHCO-R3, NHSO₂-R3, NHCONH-R3, NHSO₂NH-R3, OCO-R3, COO-R3, OSO₂-R3, SO₂O-R3, OCONH-R3, OSO₂NH-R3, dans lequel chaque R3 est indépendamment choisi parmi H, alkyle, cycloalkyle, alcènyle, aryle, hétéroaryle, hétérocyclyle, éventuellement substitué par halogène, aryle, hétéroaryle, OR4, N(R4)₂, dans lequel chaque R4 est indépendamment choisi parmi H, C1-C4 alkyle.

2. Produit selon la revendication 1, **caractérisé en ce que** R1 est hérétoaryle, éventuellement substitué.

3. Produit selon la revendication 2, **caractérisé en ce que** R1 est choisi parmi benzimidazolyle, indolyle, pyrrolyle, éventuellement substitué par halogène, R4, O-R4.

4. Produit selon la revendication 3, **caractérisé en ce que** R1 est choisi parmi benzimidazol-2-yle, indol-2-yle, pyrrol-2-yle, éventuellement substitué par halogène, R4, O-R4.

5. Produit selon l'une quelconque des revendications 1 à 4, **caractérisé en ce que** R2 est choisi parmi phényle, pyridyle, thiényle, C1-C4 alkyle, C3-C7 cycloalkyle, éventuellement substitué.

6. Produit selon l'une quelconque des revendications 1 à 5, **caractérisé en ce que** X est choisi parmi CO, SO₂.

7. Produit selon la revendication 1, **caractérisé en ce que** R1 est aryle substitué.

8. Produit selon la revendications 1, **caractérisé en ce que** X est liaison, et **en ce que** R2 est choisi parmi aryle substitué et hétéroaryle substitué.

9. Produit selon la revendication 8, **caractérisé en ce que** R2 est choisi parmi
- aryle substitué par NHSO₂-R3 ou NHCONH-R3, et
- hétéroaryle substitué par NHSO₂-R₃, ou NHCONH-R3.

10. Produit selon la revendication 9, **caractérisé en ce qu'**aryle est phényle, et **en ce que** hétéroaryle est choisi parmi pyridyle et pyrimidyle.

11. Produit selon la revendication 9, **caractérisé en ce que** R3 est choisi parmi aryle substitué et hétéroaryle substitué.

12. Produit selon la revendication 11, **caractérisé en ce que** R3 est substitué par un substituant sélectionné dans le groupe constitué par halogène, R4, OR4, N(R4)₂, dans lequel chaque R4 est indépendamment choisi parmi H, C1-C4 alkyle, et C1-C4 alkyle halogéné.

13. Produit selon l'une quelconque des revendications précédentes, **caractérisé en ce qu'**il est sous forme :
- racémique, ou
- enrichie en un stéréoisomère, ou
- enrichie en un énantiomère ;
et **en ce qu'**il est éventuellement salifié.

14. Composition pharmaceutique comprenant un produit selon l'une quelconque des revendications précédentes, en combinaison avec un excipient pharmaceutiquement acceptable.

15. Utilisation d'un produit selon l'une quelconque des revendications 1 à 13, comme agent modulant l'activité d'une kinase.

16. Utilisation d'un produit selon la revendication 15, dans laquelle la kinase est choisie parmi Tie2 et KDR.

17. Produit selon l'une quelconque des revendications 1 à 13, pour son utilisation pour traiter un état pathologique.

18. Produit selon l'une quelconque des revendications 1 à 13, pour son utilisation pour le traitement du cancer.
